# EUROPEAN PATENT SPECIFICATION

(11) **EP 4 225 930 B1**
(45) Date of publication and mention of the grant of the patent: **14.05.2025**
(21) Application number: 21785920.6
(22) Date of filing: 07.10.2021
(51) Int. Cl.: C12P 21/00, C12P 19/02, C12P 19/12, C12P 19/14, C12N 1/14, C12N 9/42

(54) **PROCESS FOR THE PRODUCTION OF A FILAMENTOUS FUNGUS WHOLE BROTH ENZYME COMPOSITION WITH LOW VISCOSITY**
VERFAHREN ZUR HERSTELLUNG EINER ENZYMZUSAMMENSETZUNG AUS FADENPILZ-VOLLBRÜHE MIT NIEDRIGER VISKOSITÄT
PROCÉDÉ DE PRODUCTION D'UNE COMPOSITION D'ENZYME DE BOUILLON COMPLET DE CHAMPIGNON FILAMENTEUX À FAIBLE VISCOSITÉ

(30) Priority: 08.10.2020 EP 20200815
(43) Date of publication of application: 16.08.2023
(73) Proprietor: Clariant Produkte (Deutschland) GmbH, 65929 Frankfurt am Main (DE)
(72) Inventor: GAMAUF, Christian, 81241 München (DE); CLAREN, Jörg, 82131 Stockdorf (DE)
(74) Representative: Clariant Produkte (Deutschland) GmbH
(86) International application number: PCT/EP2021/077659
(87) International publication number: WO 2022/074102

(56) References cited:
- WO-A1-2011/151513
- DATABASE GENBANK [online] 30 March 2017 (2017-03-30), LI W.-C. ET AL: "Trichoderma reesei QM6a chromosome VI, complete sequence TITLE Trichoderma reesei Complete Genome Sequence, Repeat-Induced Point Mutation And Partitioning Of CAZyme Gene Clusters", XP055794352, Database accession no. CP016237
- DATABASE UNIPROT [online] 7 October 2020 (2020-10-07), KOIKE H. ET AL: "Dicer-like protein 2", XP055794364, Database accession no. A0A024S2T0
- DATABASE GENBANK [online] 30 March 2017 (2017-03-30), LI, W.-C. ET AL.: "Trichoderma reesei QM6a chromosome VI, complete sequence", XP055794352, Database accession no. CP016237
- DATABASE UniProt [online] 7 October 2020 (2020-10-07), KOIKE, H. ET AL.: "Dicer-like protein 2", XP055794364, Database accession no. A0A024S2T0

## Description

The present invention relates to a process for the production of a filamentous fungus whole broth enzyme composition with low viscosity, a genetically modified filamentous fungus cell for production of the whole broth enzyme composition, the use of such a genetically modified filamentous fungus cell for the production of the filamentous fungus whole broth enzyme composition with low viscosity and a filamentous fungus whole broth enzyme composition produced by such a method.

Hydrolysate from lignocellulose-containing (or lignocellulosic) biomass is coming more and more into focus as a valuable substrate for the production of various substances such as enzymes, lactic acid, fatty acids, iso-butanol, butanediol, succinic acid, itaconic acid but also ethanol. Ethanol originating from such a process is usually referred to as "bioethanol" or "biofuel". Production of the desired substances is often carried out by fermentation processes involving yeasts, bacteria or fungi capable of producing the desired end product.

Within the WO 2011151513 a method for genetically modifying a filamentous fungus host for improved protein production is described. The method comprises that a filamentous fungus host is genetically modified to overexpress or to be deficient of specific genes. The WO 2011151513 also descriebs a method for improved production or for producing an improved composition of proteins, such as cellulases, hemicellulases, other proteins involved in the degradation of lignocellulosic material, or other proteins, in a filamentous fungus host.

Within the Database Genbank (LI, W.-C. et al.) a Trichoderma reesei QM6a chromosom VI, complete sequence, accessions no. CP016237, XP 055794352 is described (within the present invention SEQ ID NO: 1).

One major drawback of such processes is the lignocellulosic biomass substrate itself. All biomasses usually referred to as "lignocellulosic substrate" contain a considerable amount of lignin (up to 40 wt.-%), cellulose (up to 55 wt.-%) and hemicelluloses (up to 55 wt.-%). Due to its origin (wood or weed plant-derived biomass) also the structure of the cell walls is significantly different from those of most other plant species which will influence hydrolysis of the substrate. Hydrolysis can be achieved by application of chemicals such as acids but is usually carried out by digestion of enzymes which will not contaminate the hydrolysate with e.g. salts resulting from chemical treatments.

The necessary hydrolytic breakdown of those polymers involves the use of several cellobiohydrolases, endoglucanases, β-glucosidases and oxidoreductases. To avoid costly supplementation of the single specific enzymes such hydrolytic breakdown is usually carried out by applying a so called "whole broth enzyme composition" produced by a microorganism, such as a filamentous fungus, capable of producing all enzymes necessary for hydrolysis of the substrate. The resulting glucose and cello-oligosaccharides can then easily be fermented to the desired end product - such as ethanol when using ordinary baker's yeast.

To attain economic feasibility, a high yield of the desired end product but also high yield of the produced whole broth enzyme composition is a necessity. This applies in particular when the end product is bioethanol which has to compete with ordinary and cheap mineral-oil derived fuel products on the market. Producing monomeric sugars from cellulose and hemicellulose at high yields is far more difficult than deriving sugars from sugar- or starch-containing crops, e.g. sugarcane or maize (Van Dyck and Pletschke, 2012). Therefore, although the cost of lignocellulosic biomass is far lower than that of sugar and starch crops, the cost of obtaining sugars from such materials for fermentation into e.g. bioethanol has often been considered to be too high to be industrially feasible. For this reason, it is crucial to solve the problems involved in the conversion of lignocellulosic biomass to hydrolysate.

One problem is the high viscosity of the fermentation broth of the fungus, especially of a filamentous fungus, which is needed for production of the whole broth enzyme composition. In order to obtain a high yield of enzymes, a strong growth of the fungus is desired, however, a strong growth comes with a high content of fungus biomass within the fermentation broth. Fungi, which are known to consist of i.a. hyphae are known within the art as rendering any fermentation substrate into a high-viscous composition. This effect is significantly more distinct when a filamentous fungus is used which exhibits a sponge-like, slimy appearance.

High viscosity causes many problems, as the fungus needs constant oxygen supply by aeration during growth. In addition, cooling of the fermenter, especially in industrial-scale production is required. Both can only be guaranteed by constant stirring - on the one hand to distribute the air bubbles homogenously within the broth, and on the other hand to facilitate constant heat-exchange with the cooling devices. The higher the viscosity of the broth the more energy needs to be spent to realize effective stirring within the reactor. Further, more air as to be pressed into the reactor causing also higher energy consumption within the compressor and sterile-filter unit. Thus, both CAPEX and OPEX increase with increasing viscosity of the fermentation broth. An alternative measure - less cell mass production - is also not attractive for commercial production as this would always be accompanied by a lower yield of whole broth enzyme production.

As lignocellulosic biomass is a substrate which already provides several challenges any further cost increase has to be avoided when applying such processes to commercial scale production.

The inventors of the present invention have therefore set themselves the task to develop a process for the production of filamentous fungus whole broth enzyme composition with low viscosity while maintaining a high yield of enzymes within the broth.

The task has been solved by a process for production of a whole broth enzyme composition, comprising the following steps:
(a) providing a fermentation medium, originating from hydrolysis of lignocellulosic biomass, with a glucose content of from 5 to 450 g/L, a xylose content of from 2 to 300 g/L, a density of from 1 to 2 kg/L and a dry matter content of from 10 to 75 wt.-%;
(b) addition of at least one filamentous fungus cell wherein SEQ1 gene sequence SEQ ID NO:1 has been disrupted;
(c) mixing of the fermentation medium and the at least one filamentous fungus cell for a time period of from 1 minute to 10 days at a temperature of from 20 to 35 °C;
(d) obtaining a whole broth enzyme composition.

Within the present invention the term "whole broth" is to be understood to consist of or to contain a partly or completely fermented medium and may even contain components of the original medium but also any compound generated during the fermentation process. "Whole broth" may also contain part of or all of the microbial biomass of the fermentation microorganism.

Within the present invention the term "whole broth enzyme composition" is to be understood as any whole broth as defined herein containing at least one enzyme. The at least one enzyme may have been added to the whole broth, may have been part of the original fermentation medium but may also be generated during the production process according to the present invention. "Whole broth enzyme composition" may also contain a mixture of two or more of such enzymes.

Within the present invention the whole broth enzyme composition preferably contains at least one enzyme belonging to the class of hydrolases. Within a particularly preferred embodiment of the present invention, the whole broth enzyme composition contains at least one enzyme belonging to the class of hydrolases which has been produced by the at least one filamentous fungus cell. Within another also particularly preferred embodiment, the whole broth enzyme composition contains at least one enzyme belonging to the class of cellulases and at least one enzyme belonging to the class of hemicellulases which has been produced by the at least one filamentous fungus cell.

Within the present invention, the term "enzyme belonging to the class of hydrolases" is to be understood as comprising any enzyme, capable of the hydrolysis of a chemical bond. Enzymes belonging to the class of hydrolases are classified as EC 3 in the EC number classification of enzymes. According to the present invention, the term "hydrolases" comprises cellulases, hemicellulases and may also encompass pectinases, oxydases and accessory proteins.

As used within the present invention, the term "cellulase" refers to any enzyme capable of hydrolyzing cellulose polymers to shorter oligomers and/or glucose. Cellulases preferred within the whole broth enzyme composition include cellobiohydrolases (CBH) (EC 3.2.1.-), endo-1,4-β-glucanases (EG) (EC 3.2.1.4).), beta-glucosidase (EC 3.2.1.4), cellobiose hydrolase (EC 3.2.1.21), glycoside hydrolase 61 (GH61 and CBM33).

As used within the present invention, the term "hemicellulase" refers to any enzyme capable of degrading or supporting the degradation of hemicellulose. Hemicellulases preferred within the whole broth enzyme composition include β-glucanases (EC 3.2.1.-), endo-xylanases (EC 3.2.1.8), β-xylosidases (EC 3.2.1.37), acetylxylan esterase (EC 3.1.1.72), acetylgalactan esterase (3.1.1.6), acetyl mannan esterase, feruloyl esterase (EC 3.1.1.73), glucuronoyl esterase (EC 3.1.1.-), α-L-arabinofuranosidase (EC 3.2.1.55), α-arabinopyranosidase (3.2.1.-), α-galactosidase (EC 3.2.1.22), ß-galactosidase (EC 3.2.1.23), α-glucuronidases (EC 3.2.1.139), β-mannase (EC 3.2.1.78), β-mannosidases (EC 3.2.1.25), mannan 1,4-mannobiosidase (EC 3.2.1.100), arabinogalactan endo-beta-1,4-galactanase (EC 3.2.1.89), endo-beta-1,3-galactanase (EC 3.2.1.90), galactan endo-beta-1,3-galactanase (EC 3.2.1.181, glucuronoarabinoxylan endo-1,4-beta-xylanase (EC 3.2.1.136), alpha-L-fucosidase (EC 3.2.1.51), coniferin beta-glucosidase (EC 3.2.1.126), xyloglucan hydrolases (EC 3.2.1.150, 151, 155), xylan α-1 ,2-glucuronosidase (EC 3.2.1.131), endo-xylogalacturonan hydrolase (EC 3.2.1.-; GH28), α-amylase (EC 3.2.1.1 ), glucan 1 ,4-α-glucosidase (EC 3.2.1.3), galactan 1,3-galactosidase (GH43), -1,4,-endogalactanase (EC 3.5.1.89; GH53), α-rhamnosidase (EC 3.2.1.40), ß-rhamnosidase (EC 3.2.1.43).

As used within the present invention, the term "pectinase" refers to any enzyme capable of degrading or supporting the degradation of pectin. Pectinases preferred within the whole broth enzyme composition include polygalacturonases (EC 3.2.1.15, 67, 82; GH28pectin methyl esterase (EC 3.1.1.11), pectin acetyl esterase (EC 3.1.1.- ), rhamnogalacturonase (EC 3.2.1.-; GH28), rhamnogalacturonan acetylesterase (EC 3.1.1.86), rhamnogalacturonan galacturonohydrolase (EC 3.2.1.-), xylogalacturonan hydrolase (EC 3.2.1.-), pectin methylesterase (EC 3.1.1.11), beta-arabinofuranosidase (EC 3.2.1.55), beta-1 ,4-galactanase (EC 3.2.1.89), beta-1 ,3-galactanase (EC 3.2.1.90), beta-galactosidase (EC 3.2.1.23), alpha-galactosidase (EC 3.2.1.22), feruloyl acetyl esterase (EC 3.1.1.-), alpha-fucosidase (EC 3.2.1.51), (beta-fucosidase) (EC 3.2.1.38), beta-apiosidase (EC 3.2.1.-), alpha-rhamnosidase (EC 3.2.1.40), beta-rhamnosidase (EC 3.2.1.43), alpha-arabinopyranosidase (EC 3.2.1.-), beta-glucuronidase (EC 3.2.1.31), alpha-glucuronidase (EC 3.2.1.139), beta-xylosidase (EC 3.2.1.37) and alpha-xylosidase (EC 3.2.1.x).

As used within the present invention the term "accessory protein" refers to any enzyme capable of supporting cellulolytic enzyme activity. The term is well known to a person skilled in the art. Preferred accessory proteins within the whole broth enzyme composition include Expansin, Swollenin, Loosinin and CIP Proteins (EC 3.1.1.-; CE15).

As used within the present invention, the term "oxidative enzymes" refers to any enzyme capable of catalyzing an oxidation reaction. Oxidative enzymes preferred within the whole broth enzyme composition include lytic polysaccharide monooxygenase (LPMO) (AA9-11; previously GH61 and CBM33, resp.) (EC 1.14.99.53-56, 1.14.99.B10), lignin peroxidase (EC 1.11.1.14), manganese peroxidase (EC 1.11.1.13), aryl-alcohol oxidase (EC 1.1.3.7), glyoxal oxidase (EC 1.1.3.), carbohydrate oxidases (EC 1.1.3.4, 9, 10), cellobiose dehydrogenase (EC 1.1.99.18), catalase (hydrogen-peroxide oxidoreductase) (EC 1.11.1.6 or EC 1 .11.1.21 ), dye-decolorizing peroxidase (EC 1.11.1.19), laccase (EC 1.10.3.2), peroxidase (EC 1.11.1.x) and versatile peroxidase (EC 1.11.1.16).

The enzymes referenced within the present invention are classified according nomenclatures that are either based on the International Union of Biochemistry and Molecular Biology's Enzyme Nomenclature and Classification (http://www.chem.qmul.ac.uk/iubmb/enzyme/) or on Carbohydrate-Active EnZYmes (http://www.cazy.org/) database.

Within the present invention, the at least one enzyme belonging to the class of hydrolases amounts preferably to from 1 to 45 wt.-% (relative to the weight of the whole broth enzyme composition), further preferred to from 1 to 25 wt.-%, particularly preferred to from 1 to 20 wt.-%, also preferred to from 2 to 15 wt.-%, from 2 to 14 wt.- %, from 3 to 12 wt.-% and most preferred to from 5 to 11 wt.-%.

Within the present invention the term "fermentation medium originating from hydrolysis of lignocellulosic biomass" can be any medium which has been prepared by chemical, mechanical and/or enzymatic hydrolysis of lignocellulosic biomass material and preferably comprises prior mechanical and/or acidic pretreatment of the lignocellulosic biomass. Within a preferred embodiment of the inventive process, the hydrolysis has been carried out by mechanical and enzymatical hydrolysis or by sole enzymatic hydrolysis without the addition of any organic and/or inorganic acid(s). The hydrolysis of lignocellulosic biomass is known to a person skilled in the art, exemplary methods are for example described within Vishnu *et al.* 2012 (Trends in bioconversion of lignocellulose: Biofuels, platform chemicals & biorefinery concept in bioconversion of lignocellulose: Biofuels, platform chemicals & biorefinery concept.

Progress in Energy and Combustion Science, August 2012, vol. 38 (4), 522-550) and Prasad et al. 2019 (Bioethanol production from waste lignocelluloses: A review on microbial degradation potential Chemosphere Volume 231, September 2019, p. 588-60).

Within the present invention the term "lignocellulose-containing biomass" is to be understood to comprise all kind of biomass known to a person skilled in the art as comprising lignocellulose. Particularly preferred lignocellulose-containing biomass according to the present invention include wood, cereal straw such as but not limited to wheat straw, rice straw, barley stray, rye straw and oat straw, and/or husks and/or brans thereof, bagasse, oat hulls, switch grass, cellulose, raw paper pulp (obtained from pulp and paper production) and mixtures thereof. Additional components may comprise one or more of the following components: purified cellulose, pulp, milk whey or molasses. Lignocellulosic biomass which is particularly suitable for hydrolysis according to the process of the present invention is selected from the group consisting of cereal straw, cereal bran, cereal husks, wood, bagasse and mixtures thereof.

In a preferred embodiment the lignocellulose-containing material contains at least 25 wt.-%, preferably at least 40 wt.-%, more preferably at least 70 wt.-%, even more preferably at least 80 wt.-% and most preferred at least 90 wt.-% lignocellulose. It is to be understood that the lignocellulose-containing material may also comprise other compounds such as proteinaceous material, starch, sugars, such as fermentable sugars and/or non-fermentable sugars.

Within the process of the present invention, the fermentation medium contains from 5 to 450 g/L glucose and from 2 to 300 g/L xylose, wherein glucose contents from 5 to 420 g/L, from 8 to 400 g/L and from 10 to 280 g/L are preferred and wherein xylose contents from 3 to 280 g/L, from ¹3 to 270 g/L and from 4 to 260 g/L are preferred. Further preferred ranges of glucose are from 10 to 450 g/L, from 40 to 400 g/L and from 50 to 350 g/L whereas further preferred ranges of xylose are from 10 to 280 g/L, from 30 to 250 g/L and from 50 to 220 g/L. Further preferred is a ratio from glucose to xylose selected from the range of from 5 to 1, such as a ratio selected from the range of from 3 to 1, from 4.0 to 1.5, of from 3.5 to 1.5 or of from 3.0 to 1.5. Ratios selected from the range of from 2.5 to 1.0 are most preferred as a maximum of glucose and xylose have been released from the lignocellulosic biomass during hydrolyzation enabling a higher yield of whole broth enzyme composition.

The fermentation medium originating from hydrolysis of lignocellulosic biomass has a high density of from 0.90 to 2.00 kg/L, preferably of from 0.95 to 1.90 kg/L, further preferred of from 1.00 to 1.50 kg/L and most preferred of from 1.05 to 1.35 kg/L.

The fermentation medium originating from hydrolysis of lignocellulosic biomass has a dry matter content of from 10 to 75 wt.-%, preferably of from 10 to 70 wt.-%, further preferred of from 20 to 65 wt.-%, from 30 to 65 wt.-% or from 40 to 60 wt.-% whereas a dry matter content of from 10 to 20 wt.-% and from 10 to 15 wt.-% is also preferred.

The "providing" of the fermentation medium according to step (a) of the inventive process can be carried out by any method and within any means known to a person skilled in the art as suitable for the inventive process. Within a preferred embodiment the fermentation medium originating from hydrolysis of lignocellulosic biomass is provided within a batch or fed batch reactor which is preferred equipped with a stirring device and a cooling device.

Within a preferred embodiment of the inventive process, the fermentation medium has a furfural content of less than 0.5 g/L, preferably less than 0.2 g/L, further preferred less than 0.1 g/L, also preferred less than 0.05 g/L and is most preferred selected from the range of from 0.001 mg/L to 0.5 g/L or from 0.01 mg/L to 0.25 g/L.

Within another preferred embodiment of the inventive process, the fermentation medium has a hydroxymethyl furfural content of less than 0.5 g/L, preferably less than 0.2 g/L, further preferred less than 0.1 g/L, also preferred less than 0.05 g/L and is most preferred selected from the range of from 0.001 mg/L to 0.5 g/L or from 0.01 mg/L to 0.25 g/L.

Within another preferred embodiment from 0.05 to 5 wt.-% nitrogen are added during step (a) and/or (b) of the inventive process. Further preferred ranges are from 0.08 to 5 wt.-%, from 0.1 to 5 wt.-% and from 0.5 to 5 wt.-%. The nitrogen can be added in any form known to a person skilled in the art as suitable for the inventive purpose and may be added in form of ammonium sulfate, ammonia, urea or combinations thereof. The amount of nitrogen can be added by feeding or by adding the total amount to the fermentation medium at any time during step (a) and/or (b) of the inventive process. It is thereby preferred that the nitrogen is added as a 25% (wt.- /wt.) solution of ammonia or a 40 % (wt./wt.) solution of urea.

Within another preferred embodiment from 0.5 to 350 mg/L FeSO₄, MnSO₄, MgSO₄ and/or ZnSO₄ are added during step (a) and/or (b) of the inventive process. The amount of FeSO₄, MnSO₄, MgSO₄ and/or ZnSO₄ can be added by feeding or by adding the total amount to the fermentation medium at any time during step (a) and/or (b) of the inventive process. It is thereby preferred that FeSO₄ is added in an amount of from 0.5 to 35 mg/l and MgSO₄ is added in an amount of from 200 to 350 mg/l.

Within a particularly preferred embodiment of the inventive process no mono- and/or disaccharides, in particular no glucose, fructose or xylose, are added to the fermentation medium originating from hydrolysis of lignocellulosic biomass at any time during the inventive process.

Within a preferred embodiment of the inventive process the pH of the fermentation medium has been adjusted to a pH selected from the range of from pH 2.0 to pH 6.0, wherein ranges of from pH 3.0 to 5.5 and from pH 3.5 to 5.5 as well as from pH 3.5 to 5.0 are particularly preferred. The adjusting of the pH can be carried out by any means and method known to a person skilled in the art as suitable for the inventive purpose. Within the process of the present invention the pH is preferably adjusted by addition of an acid such as sulfuric acid or acetic acid, NaOH, H₃PO₄ or ammonia.

Within a preferred embodiment of the inventive process the process further comprises step (ai) concentration of the fermentation medium by evaporation, membrane filtration, thin layer evaporation, falling-film or downstream evaporation to decrease the weight of the fermentation medium by factor 2 to 6, wherein a decrease of weight of the fermentation medium by a factor of 2.5 to 6, 3 to 6, 3.5 to 6 and 4 to 6 is also preferred. The decrease of weight of the fermentation medium is thereby mostly achieved due to a decrease of water content of the original fermentation medium. It is thereby possible to achieve the decrease of weight of the fermentation medium by concentration of only part of the medium and blending the concentrated and non-concentrated medium before carrying out step (b) or by concentration of the complete fermentation medium to the desired end content, desired weight decrease, respectively.

Within a further preferred embodiment of the inventive process the fermentation medium originating from lignocellulosic biomass has a content of organic acids of less than 5 wt.-%, a content of inorganic acids of less than 6 wt.-%, a content of inorganic salts of less than 3 wt.-% and/or an arabinose content of less than 1 wt.-%, wherein the following contents are particularly preferred: 0.5 to 2.5 wt.-% organic acids, 0.5 to 5 wt.-% inorganic acids, 0.2 to 2.5 wt.-% inorganic salts and/or 0.05 to 0.75 wt.-% arabinose. It is thereby also particularly preferred that the content of water soluble chloride is below 0.5 wt.-%, the content of acetic acid is below 35 g/L and/or the content of Na-D/L-lactate is below 15 g/L.

According to step (b) of the inventive process, at least one filamentous fungus cell wherein SEQ ID NO: 1 has been disrupted is added to the fermentation medium. The addition can be carried out by any means and measure known to a person skilled in the art as suitable for the inventive process. Within a preferred embodiment, the at least one filamentous fungus cell is added in a quantity of from 10² to 10¹⁰ cells, preferably in a quantity of from 10³ to 10⁸ cells and most preferred in a quantity of from 10⁴ to 10⁷ cells per g of fermentation medium. The at least one filamentous fungus cell can thereby be added in dried form, as conidia or in form of a preculture, containing rest of preculturing medium. It is also possible to add the at least one filamentous fungus cell in form of a fully cultured medium (also referred to as main culture).

Within the present invention the term "filamentous fungus cell" is to be understood as any cell from any filamentous fungus existing in nature and/or known to a person skilled in the art. The term also comprises any filamentous fungus cell either of natural origin or modified. The term "modified" refers to genetically and non-genetically modified fungi. i.e. fungi which have been modified by genetic methods (e.g. transformation) and non-genetic methods e.g. chemical mutagenesis or irradiation, both of which are known to those skilled in the art. For example, the at least one filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes,* wherein *Trichoderma* is preferred, most preferred is *Trichoderma reesei* (teleomorph: *Hypocrea jecornia*). Within another preferred embodiment of the present invention, the at least one filamentous fungus cell is a genetically modified filamentous fungus cell with the with the ability to express at least one heterologous hydrolase enzyme, at least one heterologous pectinase enzyme, at least one heterologous oxidative enzyme and/or at least one heterologous accessory protein, preferably an enzyme belonging to the class of beta-glucosidases, to the class of xylanase enzymes, to the class of beta-xylosidase enzymes and/or to the class of lytic polysaccharide monooxygenase enzymes.

Within such a preferred embodiment, the at least one heterologous hydrolase enzyme preferably originates from another filamentous fungus such as - but not limited to *- Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.* Within a particularly preferred embodiment the at least one filamentous fungus cell is a *Trichoderma reesei* cell and the at least one heterologous hydrolase enzyme originates from *Acremonium, Ajellomyces, Alternaria, Armillaria, Arthroderma, Aspergillus, Bionectria, Bipolaris, Ceriporiopsis, Chaetomium, Cladophialophora, Clohesyomyces, Colletotrichum, Coniochaeta, Coniosporium, Diaporthe, Dothistroma, Emericella, Epicoccum, Exophiala, Fomes, Fonsecaea, Fusarium, Gibberella, Grosmannia, Hebeloma, Hortaea, Humicola, Hypocrea, Hypoxylon, Irpex, Isaria, Kuraishia, Leucoagaricus, Madurella, Magnaporthe, Marssonina, Metarhizium, Moniliophthora, Myceliophthora, Mycosphaerella, Neurospora, Oidiodendron, Ophiostoma, Paecilomyces, Paraphaeosphaeria, Penicillium, Phanerochaete, Phialophora, Pleurotus, Pochonia, Pseudocercospora, Pseudogymnoascus, Pyrenophora, Rasamsonia, Rhinocladiella, Rhizopus, Rhizosphaera, Rhynchosporium, Setosphaeria, Sphaerulina, Sporothrix, Stachybotrys, Stemphylium, Talaromyces, Termitomyces, Tilletiaria, Torrubiella, Trametes, Trichoderma, Trichophyton, Uncinocarpus* and/or Valsa species.

According to the present invention, the at least one filamentous fungus cell as is a filamentous fungus cell wherein SEQ1 gene sequence SEQ ID NO: 1 has been disrupted. The "disruption" can thereby be carried out by any means and measure known to the person skilled in the art as suitable for the purpose of disruption. The term "disruption" in particular comprises all techniques that either lead to the gene no longer being transcribed or to the protein encoded by the gene no longer being produced or only being produced in an inactive form.

Exemplary methods which can be used within the present invention are:
- the partial or complete removal from the genome of the gene, the region coding for the protein and/or the promoter or other regions necessary for the expression of the gene (= "deletion")
- the alteration of the DNA sequence of the coding region so that either a shortened protein (= generation of a stop codon) or a protein with an altered amino acid sequence is produced which can no longer perform the function of the unchanged protein (= "mutation")
- the modification of the DNA sequence of the promoter or other regions necessary for the expression of the gene, so that the gene is no longer transcribed (= no RNA and therefore no protein is produced)
- the expression of RNA with a sequence complementary to that of the target gene. This leads to hybridization (= pairing of complementary sequences) of the two RNAs and to a degradation of this double-stranded RNA. As a result, no RNA of the target gene is available for protein synthesis (= RNA interference).

Within the present invention SEQ ID NO:1 is defined within the sequence protocol.

Mixing according to step (c) of the process of the present invention is carried out for a time period from 1 minute to 10 days, preferably from 10 hours to 7 days, further preferred from 24 hours to 5 days, preferably under constant stirring with a power input from 150 to 20000 W/ m³ and more preferably from 500 to 15000 W/m³ and under oxygen controlled conditions. The average dissolved oxygen level is preferably selected from 0.01% to 80%, preferred from 0.1% to 50%, particularly preferred from 5% to 30% and most preferred from 12% to 28%. Within a particularly preferred embodiment, the dissolved oxygen level is controlled by a stirrer or compressed air flow or internal reactor pressure or a combination of two or three of these measures. Furthermore, mixing according to step (c) of the inventive process is carried out at a temperature of from 20 to 35 °C, preferably at a temperature of from 21 to 34 °C wherein a temperature selected from the range of from 22 to 33 °C is also preferred.

"Mixing" according to step (c) of the process of the present invention is preferably conducted in a batch mode (discontinuous), in the fed-batch mode or in a continuous mode. Most preferably, the inventive process is conducted in the batch mode.

"Obtaining" according to step (d) of the inventive process is preferably carried out by harvesting the whole fermentation broth at the end of the time period applied for mixing during step (c) as it is without further treatment. It is, however, also possible within an alternative embodiment to practice a solid-liquid-separation according to step (e) of the inventive process. The solid-liquid-separation according to step (e) is carried out by any measure known to a person skilled in the art as suitable for the inventive purpose such as but not limited to filtration, pressing, membrane separation, flotation, precipitation, decantation and centrifugation or combinations thereof. Preferred are filter-based solid-liquid separations. It is further particularly preferred to use a filter press. The residues after the filtration should have a minimal solid content of 20 % (wt./wt.), preferably 25 % (wt./wt.), particularly preferred 30 % (wt./wt.) and most preferred 40 % (wt./wt.) solid content. Another method for the separation according to step (e) is centrifugation by e.g. using a decanter. In case the process according to the present invention involves solid-liquid-separation, the whole broth enzyme composition obtained according to step (d) of the inventive process is considered to be the liquid fraction.

Within a preferred embodiment of the inventive process, the process further comprises step
(aii) sterilization of the fermentation medium according to step (a) or the concentrated fermentation medium according to step (ai).

Sterilization can thereby be carried out by any means or measure known to a person skilled in the art as suitable for the inventive purpose. Within a preferred embodiment, sterilization is carried out by filtration, such as but not limited to membrane filtration processes or by ultra high temperature heating. A combination of two or more sterilization methods is also possible, however, it is particularly preferred to only apply ultra high temperature heating (also referred to as UHT). The UHT treatment is preferably carried out at a temperature of from 100 to 155 °C and for a duration of from 10 to 30 seconds, more preferred at a temperature of from 120 to 140 °C for a duration of from 10 to 20 seconds.

Within another aspect, the present invention relates to a filamentous fungus cell wherein SEQ ID NO:1 has been disrupted. Disruption of SEQ ID NO:1 can be carried out by any means and measure known to a person skilled in the art to be suitable for the inventive purpose. Possible and preferred methods and measures have been defined within the description. Within a preferred embodiment, SEQ ID NO:1 has been disrupted by deletion, mutation, modification of a promotor or any other regulatory sequence, generation of a stop codon or RNA interference. The term "filamentous fungus cell" has been defined within the description. All definitions given apply.

Within a preferred embodiment, the filamentous fungus cell is a genetically modified filamentous fungus cell with the ability to express at least one heterologous hydrolase enzyme. Within a particularly preferred embodiment, the filamentous fungus cell contains at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence. The respective heterologous enzyme sequence may originate from any fungus or microorganism known to a person skilled in the art as suitable for the inventive purpose. Within a preferred embodiment the heterologous enzyme sequence originates from another species of filamentous fungus.

In another aspect the present invention relates to a whole broth enzyme composition prepared according to the process as defined before.

In a further aspect the present invention relates to the use of a whole broth enzyme composition as defined before for the hydrolyzation of lignocellulosic biomass. The definition within the description for "hydrolyzation" and "lignocellulosic biomass" apply.

### Generally preferred embodiments

In the following, generally preferred embodiments of the present invention are listed. The generally preferred embodiments illustrate particularly suitable embodiments for the production of whole broth enzyme composition by the filamentous fungus *Trichoderma reesei.*

### Generally preferred embodiment A

Process for production of a whole broth enzyme composition, comprising the following steps:
(a) providing a fermentation medium, originating from hydrolysis of lignocellulosic biomass, with a glucose content of from 40 to 400 g/L, a xylose content of from 50 to 200 g/L, a density of from 1.05 to 1.35 kg/L and a dry matter content of from 30 to 65 wt.-%;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment B

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 40 to 400 g/L, a xylose content of from 50 to 200 g/L, a density of from 1.05 to 1.35 kg/L, a dry matter content of from 30 to 65 wt.-%, a nitrogen content of from 0.05 to 5 wt.-%, a MgSO4 content of from 200 to 350 mg/L and a average dissolved oxygen level of from 5 to 30%;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment C

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 40 to 400 g/L, a xylose content of from 50 to 200 g/L, a density of from 1.05 to 1.35 kg/L, a dry matter content of from 30 to 65 wt.-%, a nitrogen content of from 0.05 to 5 wt.-%, a MgSO4 content of from 200 to 350 mg/L, a average dissolved oxygen level of from 5 to 30% and a pH of from 3.5 to 5;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition containing at least one enzyme belonging to the class of cellulases and at least one enzyme belonging to the class of hemicellulases which has been produced by the at least one *Trichoderma reesei* cell.

### Generally preferred embodiment D

Process for production of a whole broth enzyme composition, comprising the following steps:
(a) providing a fermentation medium, originating from hydrolysis of lignocellulosic biomass, with a glucose content of from 5 to 25 g/L, a xylose content of from 2 to 15 g/L, a density of from 1.05 to 1.35 kg/L and a dry matter content of from 30 to 65 wt.-%;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment E

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 5 to 25 g/L, a xylose content of from 2 to 15 g/L, a density of from 1.05 to 1.35 kg/L, a dry matter content of from 30 to 65 wt.-%, a nitrogen content of from 0.05 to 5 wt.-%, a MgSO4 content of from 200 to 350 mg/L and a average dissolved oxygen level of from 5 to 30%;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment F

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 5 to 25 g/L, a xylose content of from 2 to 15 g/L, a density of from 1.05 to 1.35 kg/L, a dry matter content of from 30 to 65 wt.-%, a nitrogen content of from 0.05 to 5 wt.-%, a MgSO4 content of from 200 to 350 mg/L, a average dissolved oxygen level of from 5 to 30% and a pH of from 3.5 to 5;
(b) addition of at least one *Trichoderma reesei* cell wherein SEQ ID NO:1 has been disrupted by deletion, mutation, modification of a promotor or any other regulatory sequence, generation of a stop codon or RNA interference;
(c) mixing of the fermentation medium and the at least one *Trichoderma reesei* cell for a time period of from 1 min to 10 days at a temperature of from 20 to 35 °C;
(d) obtaining a whole broth enzyme composition containing at least one enzyme belonging to the class of cellulases and at least one enzyme belonging to the class of hemicellulases which has been produced by the at least one *Trichoderma reesei* cell.

### Generally preferred embodiment G

Process for production of a whole broth enzyme composition as defined by any of generally preferred embodiments A to F, wherein the *Trichoderma reesei* cell is further genetically modified by genetic methods (e.g. transformation) and/or non-genetic methods e.g. chemical mutagenesis or irradiation and wherein this further genetically modified *Trichoderma reesei* cell is able to express at least one heterologous hydrolase enzyme, at least one heterologous pectinase enzyme, at least one heterologous oxidative enzyme and/or at least one heterologous accessory protein.

### Generally preferred embodiment H

*Trichoderma reesei* cell wherein SEQ ID NO:1 has been disrupted by deletion, mutation, modification of a promotor or any other regulatory sequence, generation of a stop codon or RNA interference and wherein the *Trichoderma reesei* cell is a genetically modified *Trichoderma reesei* cell, wherein the *Trichoderma reesei* cell comprises at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence.

### Generally preferred embodiment I

*Trichoderma reesei* cell as defined by generally preferred embodiment H, wherein the at least one heterologous beta-glucosidase enzyme encoding sequence originates from *Cladophialophora* species, *Pseudocercospora* species and/or *Talaromyces* species and wherein the at least one xylanase enzyme encoding sequence originate from *Fomes* species, wherein the at least one beta-xylosidase encoding enzyme sequence originates from *Aspergillus* species and wherein the at least one lytic polysaccharide monooxygenase enzyme encoding sequence originates from *Aspergillus* species, *Trichoderma* species or *Hypocrea* species.

### Generally preferred embodiment J

Whole broth enzyme composition produced according to a process as defined by any of generally preferred embodiments A to G containing at least one heterologous betaglucosidase enzyme produced by the genetically modified *Trichoderma reesei* cell as defined by generally preferred embodiment H or I and containing whole of or part of these *Trichoderma reesei* cell.

### Generally preferred embodiment K

Use of a geneticall modified Trichoderma reesei cell as defined by generally preferred embodiment H or I for the production of whole broth enzyme composition as defined by generally preferred embodiment J for the hydrolyzation of lignocellulosic biomass.

### Figures and examples

The present invention is described by the following figures and examples. It is thereby emphasized that the figures and examples constitute further illustration of the invention, inventive purpose and benefits achieved by the inventive method.

### List of figures

- Figure 1:: Protein concentrations in the culture supernatants of pSEQ1D transformants DSEQ1-1 to -3 and reference strain M18.2b. Values are given in relation to the average protein concentration in the supernatants of the host strain M18.2b which is set to 1.
- Figure 2:: Biomass concentrations in the culture supernatants of pSEQ1D transformants DSEQ1-1 to -3 and reference strain M18.2b. Values are given in relation to the average biomass concentration in the supernatant of the host strain M18.2b which is set to 1.
- Figure 3:: Viscosity of culture broths of pSEQ1D transformants DSEQ1-1 to -3 and reference strain M18.2b. Values are given in relation to the viscosity of the culture broth of the host strain M18.2b which is set to 1.
- Figure 4:: SDS-PAGE gel of culture supernatants of pSEQ1D transformants DSEQ1-1 to -3 and reference strain M18.2b. Reference bands ("Marker" lane) correspond to 250, 150, 100, 75, 50, 37, 25 and 20 kD
- Figure 5:: Protein concentrations in the culture supernatants of pSEQ1M1-HygR transformants M1SEQ1-1 to -3 and reference strain M18.2b. Values are given in relation to the average protein concentration in the supernatants of the host strain M18.2b which is set to 1.
- Figure 6:: Biomass concentrations in the culture supernatants of pSEQ1M1-HygR transformants and M1SEQ1-1 to -3 and reference strain M18.2b. Values are given in relation to the average biomass concentration in the supernatant of the host strain M18.2b which is set to 1.
- Figure 7:: Viscosity of culture broths of pSEQ1M1-HygR transformants M1SEQ1-1 to -3 and reference strain M18.2b. Values are given in relation to the viscosity of the culture broth of the host strain M18.2b which is set to 1.
- Figure 8:: SDS-PAGE gel of culture supernatants of pSEQ1M1-HygR transformant M1SEQ1-1 to -3 and reference strain M18.2b. Reference bands ("Marker" lane) correspond to 250, 150, 100, 75, 50, 37, 25 and 20 kD
- Figure 9:: Protein concentrations in the culture supernatants of pSEQ1M2-HygR transformant M2SEQ1-1 and reference strain M18.2b. Values are given in relation to the average protein concentration in the supernatants of the host strain M18.2b which is set to 1.
- Figure 10:: Biomass concentrations in the culture supernatants of pSEQ1M2-HygR transformant M2SEQ1-1 and reference strain M18.2b. Values are given in relation to the average biomass concentration in the supernatant of the host strain M18.2b which is set to 1.
- Figure 11:: Viscosity of culture broths of pSEQ1M2-HygR transformant M2SEQ1-1 and reference strain M18.2b. Values are given in relation to the viscosity of the culture broth of the host strain M18.2b which is set to 1.
- Figure 12:: SDS-PAGE gel of culture supernatants of pSEQ1M2-HygR transformant M2SEQ1-1 and reference strain M18.2b. Reference bands ("Marker" lane) correspond to 250, 150, 100, 75, 50, 37, 25 and 20 kD

### General

The examples describe three different ways to inactivate the *Trichoderma reesei* SEQ1 gene - the deletion of a large part of the coding sequence, a mutation that changes an early codon to a stop codon and an insertion resulting in a frame shift - and show the effect of the SEQ1 gene inactivation on the protein production, biomass formation and culture broth viscosity of T. *reesei.*

### Example 1: Deletion of SEQ1

### Construction of a SEQ1 deletion vector

Standard methods known to those skilled in the art and described e.g. by Sambrook and Russel (Molecular Cloning - A laboratory manual; Cold Spring Harbor Laboratory Press, New York) or by Jansohn et al. (Gentechnische Methoden, Elsevier, München) were used for DNA agarose gel electrophorese, purification of DNA, transformation of *Escherichia coli,* plasmid propagation and purification, amplification of pieces of DNA by polymerase chain reaction (PCR) and isolation of genomic DNA from *Trichoderma reesei.* Ligation-independent cloning (LIC) was done essentially as described by Aslanidis and de Jong (1990, Nucleic Acid Res. 18 (20), 6069).

The SEQ1 5' flanking region was amplified by PCR using genomic DNA from *Trichoderma reesei* M18.2b (DSM 19984) as template, primers SEQ1 D5fw (5'-GACTCTCTATCTGCATCAAC -3') and SEQ1D5rv (5'-TGACCTGGAAAGCTTTCAATGTAGAGGTAGACTAGTCAAAGAAGACATCACGAC -3') and phusion polymerase from Thermo Fisher Scientific according to the manufacturer's instructions (annealing temperature: 64.8 °C, elongation time: 1 min 25 sec, 30 cycles). The amplicon (2.7 kb) was purified using the Wizard PCR purification kit from Promega.

The SEQ1 3' flanking region was amplified by PCR using genomic DNA from *Trichoderma reesei* M18.2b (DSM 19984) as template, primers SEQ1D3fw (5'-CGCATGGTGGGCGTCGTGATGTCTTCTTTGACTAGTCTACCTCTACATTGAAAG C -3') and SEQ1D3rv (5'- GATTACCTGTCAAGTCTATG -3') and phusion polymerase from Thermo Fisher Scientific according to the manufacturer's instructions (annealing temperature: 62.4 °C, elongation time: 1 min 25 sec, 30 cycles). The amplicon (2.7 kb) was purified using the Wizard PCR purification kit from Promega.

The SEQ1 5' and 3' flanking regions were fused by PCR using Phusion polymerase (Thermo Fisher Scientific) and the buffer and dNTP solution provided with the polymerase. 100 ng purified SEQ1 5' PCR amplicon, 100 ng purified SEQ1 3' amplicon, 10 µl 5x Phusion HF buffer, 1 µl 10 mM dNTP solution, 1 U Phusion polymerase and PCR grade water up to a final volume of 48 µl were mixed. The mixture was first incubated at 98 °C for 30 °C and then subjected to 10 cycles of 10 sec at 98 °C, 30 sec at 65 °C and 2 min 40 sec at 72 °C and then cooled to 10 °C. Then 1 µl of a 20 µM solution of primer SEQ1Dnestfw (5'-GACAGTCCTGCAGGAGTCACTGCCTTTGAAAG -3') and 1 µl of a 20 µM solution of primer SEQ1Dnestrv (5'- GACAGTCCTGCAGGTGTAAGGATAAAGGACGAC -3') were added and the mixture was incubated at 98 °C for 30 sec and then subjected to 30 cycles of 10 sec at 98 °C, 30 sec at 66.2 °C and 1 min 20 sec at 72 °C. The incubation time at 72 °C was increased by 5 sec per cycle. Finally, the mixture was incubated at 72 °C for 10 min and then cooled to 10 °C. The amplicon (5.2 kb) was purified using the Wizard PCR purification kit from Promega.

The purified *SEQ1* 5'-3' flank fusion product was digested with *Sbfl* (New England Biolabs) according to the manufacturer's instructions and purified using the Wizard PCR purification kit from Promega.

Plasmid pUC19 (New England Biolabs) was digested with *Sbfl* (New England Biolabs) according to the manufacturer's instructions and purified using the Wizard PCR purification kit from Promega.

The *Sbf*l-digested *SEQ1* 5'-3' flank fusion product and pUC19 were ligated using the "Mighty Mix" DNA ligation kit (Takara) according to the manufacturer's instructions using a molar insert/vector ratio of 5 to 1. The ligation mixture was transformed into Escherichia coli Mach 1 (Thermo Fisher Scientific) and plated on LB agar plates containing 100 mg·l⁻¹ ampicillin. After 20 h of incubation at 37 °C colonies were picked from the plate and used to inoculate 3 ml of LB liquid medium with 100 mg·l⁻¹ ampicillin. After 20 h of incubation at 37 °C plasmid DNA was isolated and digested with *Sbfl* to identify clones containing the insert. A plasmid containing the insert was designated pSEQ1-5'-3'.

Plasmid pSEQ1-5'-3' was digested with *Spe*I (New England Biolabs) according to the manufacturer's instructions and purified using the Wizard PCR purification kit from Promega. 1 µl each of 10 µM solutions of oligonucleotides LIC1fw (5'-CTAGGAGTTCTGCCTTGGGTTTAAACGAGAGAAAGACTC -3') and LIC1rv (5'-CTAGGAGTCTTTCTCTCGTTTAAACCCAAGGCAGAACTC -3') were mixed, put in a PCR cycler and cooled from 70 to 20 °C over the course of 2 h. Then the oligonucleotide mixture was mixed with 750 ng of purified, *Spe*I-digested pSEQ1-5'-3', 1 µl 10x T4 Ligase buffer (Promega), 1 µl 500 g/l PEG3350, 1 µl T4 DNA Ligase (5 U/µl; Thermo Fisher Scientific) and 2 µl of PCR-grade water. The mixture was incubated for 1 h at 20 °C, purified using the Wizard PCR purification kit from Promega and the DNA eluted in 50 µl of PCR-grade water. This solution was supplemented with 6 µl of Taq Polymerase buffer (Promega) and PCR-grade water was added to a final volume of 60 µl. The mixture was then transformed into Escherichia coli Mach 1 (Thermo Fisher Scientific) and plated on LB agar plates containing 100 mg·l⁻¹ ampicillin. After 20 h of incubation at 37 °C colonies were picked from the plate and used to inoculate 3 ml of LB liquid medium with 100 mg·l⁻¹ ampicillin. After 20 h of incubation at 37 °C plasmid DNA was isolated and digested with *Pmel* and *Ssp*I (New England Biolabs) according to the manufacturer's instructions to identify clones containing the insert. A plasmid containing the insert was designated pSEQ1-5'-3'-LIC.

Plasmid pSEQ1-5'-3'-LIC was digested with *Pme*I (New England Biolabs) according to the manufacturer's instructions and purified using the Wizard PCR purification kit from Promega.

The hygromycin B resistance cassette (HygR) (SEQ ID NO: 4) was synthesized by Thermo Scientific. HygR was amplified by PCR using the DNA from Thermo Scientific as template, primers SEQ1MHygRfw (5'-GTTCTGCCTTGGGTTTAACAAGACACAGCCCTATAAC -3') and SEQ1MHygRrv (5'- GTCTTTCTCTCGTTTAACAGACAAGAGCCCTATAAC -3') and phusion polymerase from Thermo Fisher Scientific according to the manufacturer's instructions (annealing temperature: 68.5 °C, elongation time: 40 sec, 30 cycles). The amplicon (2.4 kb) was purified using the Wizard PCR purification kit from Promega.

The PCR-amplified HygR was fused with *Pme*I-digested pSEQ1-5'-3'-LIC using ligation independent cloning (LIC). The linearized vector was treated with T4 DNA polymerase in the presence of dATP. PCR-amplified HygR was treated with T4 DNA polymerase in the presence of dTTP. T4 DNA polymerase treated vector and HygR were mixed and annealed as described by Aslanidis and de Jong (1990, Nucleic Acid Res. 18 (20), 6069). The assays were then transformed in chemically competent *Escherichia coli* Mach 1 (Thermo Fisher Scientific), plated on LB-Agar plates containing 100 mg·l⁻¹ ampicillin and incubated at 37 °C for 24 h. Colonies were picked from the agar plates using toothpicks, transferred into liquid LB medium containing 100 mg·l⁻¹ ampicillin and incubated at 37 °C for 24 h with shaking (250 RPM). Plasmid DNA was isolated and integration of the insert was verified by digestion with *Sbf*I. Plasmid clones were verified by Sanger sequencing and one plasmid with correct sequence was designated pSEQ1D.

### Transformation of the SEQ1 deletion vector into Trichoderma reesei

Vector pSEQ1D was digested with *Sbf*I (New England Biolabs) according to the manufacturer's instructions and the deletion cassette (7.8 kb) was purified by agarose gel electrophoresis and with the Wizard PCR purification kit from Promega. *Trichoderma reesei* M18.2b (DSM 19984) was transformed with the digested vector essentially as described in Penttilä et al (1987) Gene 61: 155-164 or Gruber et al (1990) Curr Genet 18: 71-76. The transformants were selected on potato dextrose agar plates containing 100 mg·l⁻¹ of hygromycin and 1 M sorbitol and purified by singularisation. Conidia stocks of the purified strains were prepared by growing them on potato dextrose agar plates at 30 °C until the plates were covered with spores. The conidia were harvested with sterile sodium chloride (0.9 g·l⁻¹)-Triton X-100 (0.01 g·l⁻¹) solution, adjusted to OD₆₀₀ = 10, supplemented with 50 g·l⁻¹ of glycerol and stored at -80 °C.

Genomic DNA was isolated from the mycelium of the transformants and the host strain. The integration of the *SEQ1* deletion cassette at the intended locus was verified by PCR using phusion polymerase from Thermo Fisher Scientific according to the manufacturer's instructions, genomic DNA from the transformants as template and primers SEQ1DKO1fw (5'- ACTCTCTATCTGCATCAAC -3') and SEQ1DKO1rv (5'- GTAGTGTATTGACCGATTC -3') (annealing temperature: 62.6 °C, elongation time: 1 min 20 sec, 30 cycles) and primers SEQ1DKO2fw (5'-TGATGTGCTTGATATTGGGC -3') and SEQ1DKO2rv (5'-CTCCATCGCTCAACTATGTG -3') (annealing temperature: 57.5 °C, elongation time: 1 min 15 sec, 30 cycles). A 3.9 kb band with primers SEQ1DKO1fw and SEQ1DKO1rv indicates the integration of the deletion cassette at the *SEQ1* locus, while SEQ1DKO2fw and SEQ1DKO2rv (1.2 kb amplicon) amplify a part of the *SEQ1* gene replaced by pSEQ1D and therefore only give a band when the SEQ1 gene is still present. Genomic DNA from strain M18.2b was also tested as a control.

Three strains that had integrated the deletion cassette from pSEQ1D at the SEQ1 locus were named DSEQ1-1 to -3.

### Growth of the SEQ1 deletion strains in shake flasks

The strains DSEQ1-1 to -3 and M18.2b were grown in shake flasks in Hydrolysate Medium 1. Hydrolysate Medium 1 contains (g·l⁻¹):

| Name | Concentration [g/L] |
|---|---|
| Acetic acid | 0.34 |
| Calcium | 0.12 |
| Chloride, water soluble | 0.15 |
| Copper | 0.0001 |
| Fat (HCl soluble) | 0.001 |
| Furfural | 0.003 |
| Glucose | 6.5 |
| Glycerol | 0.009 |
| HMF | 0.006 |
| Iron | 0.004 |
| Magnesium | 0.048 |
| Manganese | 0.002 |
| Na-D/L-Lactat | 0.097 |
| Nitrogen, soluble | 0.85 |
| Phosphorus | 0.48 |
| Phthalate | 8.2 |
| Potassium | 3.2 |
| Sodium | 0.015 |
| Sulfur | 0.86 |
| Xylose | 3.6 |
| Zinc | 0.001 |

The medium was adjusted to pH 5.5 with HCl or NaOH and sterilized by autoclaving (20 min at 121 °C).

15 ml of the medium were distributed into 50 ml Erlenmeyer shake flasks under a sterile hood. Conidia stocks of strains DSEQ1-1 to -3 and M18.2b were thawed, 75 µl of the conidia suspensions were pipetted into the Erlenmeyer flasks with the medium under a sterile hood and the flasks were closed with rubber foam caps. Three flasks were inoculated per strain. The flasks were incubated at 30 °C with shaking (250 RPM) for 6 days. After 6 days, the cultures were poured into 15 ml tubes. Aliquots were removed, centrifuged (3220xg, 4 °C, 15 min) and the supernatants stored at 4 °C, while the remaining culture broth was used for determination of the biomass and viscosity (see below).

### Characterization of the culture supernatants and broths: Protein concentration, SDS-PAGE, Biomass, Viscosity

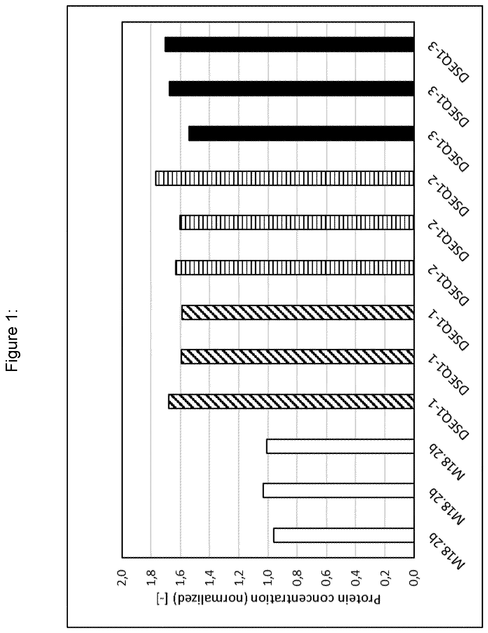

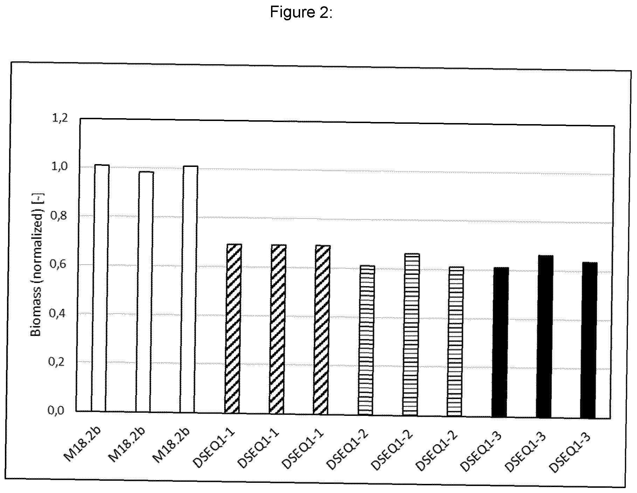

Protein concentrations in the centrifuged culture supernatants of strains DSEQ1-1 to -3 and M18.2b were measured using the Quick Start^{™} Bradford reagent (BioRad) and BSA standard solutions (BioRad) according to the supplier's instructions. The results of the measurements are shown in Figure 1. It is obvious from these data that strains DSEQ1-1 to -3 produce significantly more protein than the host strain M18.2b. For biomass determination, Whatman^{™} filter discs (P1) were dried at 60 °C until their weight remained constant for 24 h. Culture broths of strains DSEQ1-1 to -3 and M18.2b were filtered using those dried filter discs and the mycelia were washed with at least ten times the broth's volume of deionized water. Then the filter discs with the mycelium were dried at 60 °C until their weight remained constant for 24 h. The filter discs with the dried mycelia were weighed. The biomass concentration in the culture broth was then calculated by subtracting the mass of the dried filter disc from the mass of the dried filter disc with the mycelia and then dividing that value by the volume of the culture broth that had been filtered. The results of the measurements are shown in Figure 2. It is obvious from these data that strains DSEQ1-1 to -3 produce significantly less biomass than the host strain M18.2b.

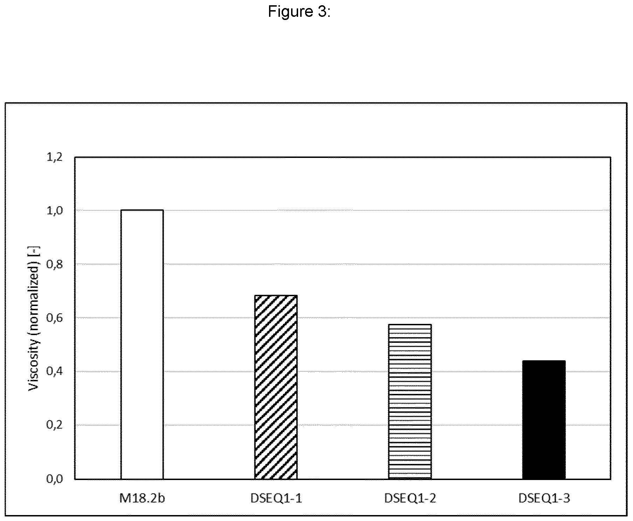

The viscosity of the culture broths of strains DSEQ1-1 to -3 and M18.2b was measured using a Malvern Kinexus Lab+ KNX2110 rotational rheometer with the Vane tool (4Vnn:CUPnn) according to the manufacturer's instructions. The measurements were taken at a temperature of 20 °C and at a rotation velocity of 18.11 RPM ("rotations per minute"). The viscosity is depicted in Figure 3 and is presented in relation to the viscosity of the culture broth of strain M18.2b, which is set to 1. It is obvious from these data that the viscosity of the culture broths produced with DSEQ1-1 to -3 is significantly lower than that of the host strain M18.2b.

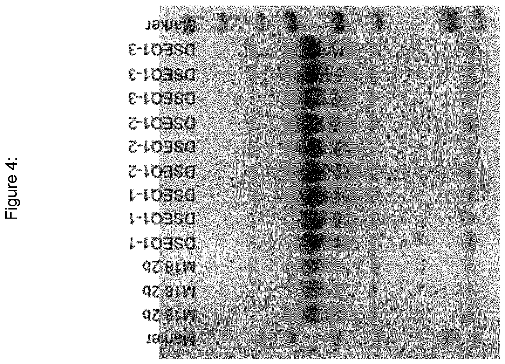

SDS-PAGE analysis of the centrifuged culture supernatants of strains DSEQ1-1 to -3 and M18.2b was done using methods known to those skilled in the art (e.g. described by Jansohn et al. (Gentechnische Methoden, Elsevier, München)) and the Criterion XT system (BioRad). Equal volumes of culture supernatants were loaded in each lane. Precision Plus Protein^{™} All Blue Standards (BioRad) was used as protein size reference. The gel image is shown in Figure 4. A person skilled in the art will recognize that the protein pattern of the *SEQ1* deletion strains DSEQ1-1 to -3 is indistinguishable from that of the host strain M18.2b.

### Summary

Taken together these data demonstrate that the deletion of the *SEQ1* gene results in a significantly more efficient protein production, with more protein and less biomass being formed. The analysis of the secreted proteins by SDS-PAGE shows that their composition doesn't change significantly, indicating a general increase in protein production. In addition, the viscosity of the culture broth is significantly reduced as well.

### Example 2: Mutation of the SEQ1 gene (early stop codon)

### Construction of a SEQ1 mutation vector

SEQ ID NO: 2, containing the flanking regions that introduce the mutation G174T (position according to SEQ ID NO: 1) into the *SEQ1* gene, and a LIC site for insertion of the marker gene was synthesized by Thermo Fisher Scientific and cloned into a pUC19-derived plasmid. The resulting plasmid was named pSEQ1M1

Plasmid pSEQ1M1 was digested with *Srf*I (New England Biolabs) according to the manufacturer's instructions and purified using the Wizard PCR purification kit from Promega.

The hygromycin B resistance cassette (HygR) (SEQ ID NO: 4) was synthesized by Thermo Scientific. HygR was amplified by PCR using the DNA from Thermo Scientific as template, primers SEQ1MHygRfw (5'- AACAAGACACAGCCCTATAAC -3') and SEQ1MHygRrv (5'- AACAGACAAGAGCCCTATAAC -3') and phusion polymerase from Thermo Fisher Scientific according to the manufacturer's instructions (annealing temperature: 68.5 °C, elongation time: 40 sec, 30 cycles). The amplicon (2.4 kb) was purified using the Wizard PCR purification kit from Promega.

The PCR-amplified HygR marker was fused with linearized pSEQ1M1 using ligation independent cloning (LIC). The linearized vector was treated with T4 DNA polymerase in the presence of dTTP. The amplified promotors were treated with T4 DNA polymerase in the presence of dATP. T4 DNA polymerase treated vector and promotors were mixed and annealed as described in Ref. The assays were then transformed in chemically competent *Escherichia coli* XL1-Blue cells (Agilent), plated on LB-Agar plates containing 100 mg·l⁻¹ ampicillin (LB-Amp) and incubated at 37 °C for 24 h. Colonies were picked from the agar plates using toothpicks, transferred into liquid LB-Amp medium and incubated at 37 °C for 24 h with shaking (250 RPM). Plasmid DNA was isolated and integration of the insert was verified by digestion with *Xmn*I*.* Plasmid clones were verified by Sanger sequencing and one plasmid with correct sequence was designated pSEQ1M1-HygR.

### Transformation of the SEQ1 mutation vector into Trichoderma reesei

Vector pSEQ1M1-HygR was digested with *Xmn*I (New England Biolabs) according to the manufacturer's instructions and the mutation cassette (6.0 kb) was purified by agarose gel electrophoresis and with the Wizard PCR purification kit from Promega. *Trichoderma reesei* M18.2b (DSM 19984) was transformed with the digested vector essentially as described in Penttilä et al. (1987) Gene 61: 155-164 or Gruber et al (1990) Curr Genet 18: 71-76. The transformants were selected on potato dextrose agar plates containing 100 mg·l⁻¹ of hygromycin and 1 M sorbitol and purified by singularisation. Conidia stocks of the purified strains were prepared by growing them on potato dextrose agar plates at 30 °C until the plates were covered with spores. The conidia were harvested with sterile sodium chloride (0.9 g·l⁻¹)-Triton X-100 (0.01 g·l⁻¹) solution, adjusted to OD₆₀₀ = 10, supplemented with 50 g·l⁻¹ of glycerol and stored at -80 °C.

Genomic DNA was isolated from the mycelium of the transformants and the host strain. The integration of the *SEQ1* mutation cassette at the intended locus was verified by PCR using phusion polymerase from Thermo Fisher Scientific according to the manufacturer's instructions, genomic DNA from the transformants as template and primers SEQ1M1KOfw (5'- GATGGCTGTGTAGAAGTAC -3') and SEQ1M1KOrv (5'- ATGAATAGGAGTGTGTGTG -3') (annealing temperature: 62.0 °C, elongation time: 1 min 25 sec, 30 cycles). A 2.5 kb band with primers SEQ1M1KOfw and SEQ1M1KOrv indicates the integration of the mutation cassette at the *SEQ1* locus. Genomic DNA from strain M18.2b was also tested as a control. In order to verify that the intended mutation had been inserted into the SEQ1 ORF, the respective region was amplified by PCR using phusion polymerase from Thermo Fisher Scientific according to the manufacturer's instructions, genomic DNA from the transformants as template and primers SEQ1M1Seqfw (5'- ATACTCGTCAACTCCATC -3') and SEQ1M1Seqrv (5'- ATCGCTCAACTATGTGAC -3') (annealing temperature: 56.3 °C, elongation time: 50 sec, 30 cycles). The 1.5 kb amplicon was purified using the Wizard PCR purification kit from Promega and sequenced using Primer M1Seq-01 (5'- AGCAAGTCAAAGTCATGAGG -3').

Three strains containing the mutation from pSEQ1M1-HygR in the *SEQ1* ORF were named M1SEQ1-1 to -3.

### Growth of the SEQ1 mutation strains in shake flasks

The strains M1SEQ1-1 to -3 and M18.2b were grown in shake flasks in Hydrolysate Medium 1 as defined before for example 1. The medium was adjusted to pH 5.5 with HCl or NaOH and sterilized by autoclaving (20 min at 121 °C).

15 ml of the medium were distributed into 50 ml Erlenmeyer shake flasks under a sterile hood. Conidia stocks of strains M1SEQ1-1 to -3 and M18.2b were thawed, 75 µl of the conidia suspensions were pipetted into the Erlenmeyer flasks with the medium under a sterile hood and the flasks were closed with rubber foam caps.

Three flasks were inoculated per strain. The flasks were incubated at 30 °C with shaking (250 RPM) for 6 days. After 6 days, the cultures were poured into 15 ml tubes. Aliquots were removed, centrifuged (3220xg, 4 °C, 15 min) and the supernatants stored at 4 °C, while the remaining culture broth was used for determination of the biomass and viscosity (see below).

### Characterization of the culture supernatants and broths: Protein concentration, SDS-PAGE, Biomass, Viscosity

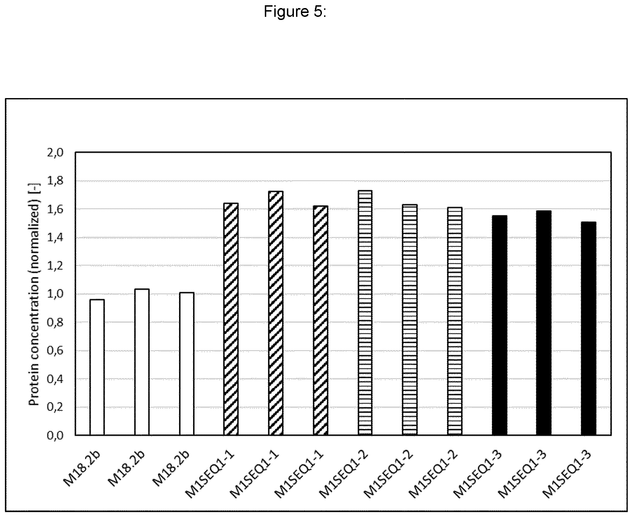

Protein concentrations in the centrifuged culture supernatants of strains M1SEQ1-1 to -3 and M18.2b were measured using the Quick Start^{™} Bradford reagent (BioRad) and BSA standard solutions (BioRad) according to the supplier's instructions. The results of the measurements are shown in Figure 5. It is obvious from these data that strains M1SEQ1-1 to -3 produce significantly more protein than the host strain M18.2b.

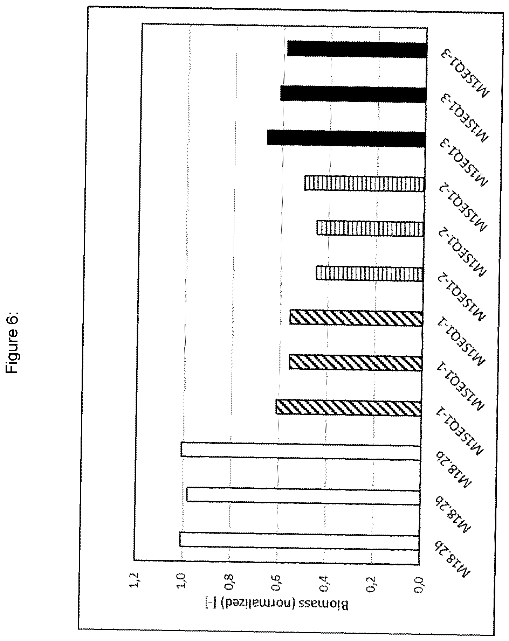

For biomass determination, Whatman^{™} filter discs (P1) were dried at 60 °C until their weight remained constant for 24 h. Culture broths of strains M1SEQ1-1 to -3 and M18.2b were filtered using those dried filter discs and the mycelia were washed with at least ten times the broth's volume of deionized water. Then the filter discs with the mycelium were dried at 60 °C until their weight remained constant for 24 h. The filter discs with the dried mycelia were weighted. The biomass concentration in the culture broth was then calculated by subtracting the mass of the dried filter disc from the mass of the dried filter disc with the mycelia and then dividing that value by the volume of the culture broth that had been filtered. The results of the measurements are shown in Figure 6. It is obvious from these data that strains M1SEQ1-1 to -3 produce significantly less biomass than the host strain M18.2b.

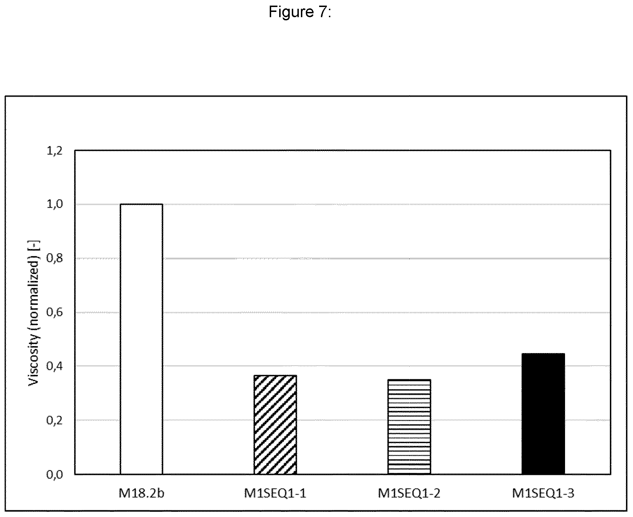

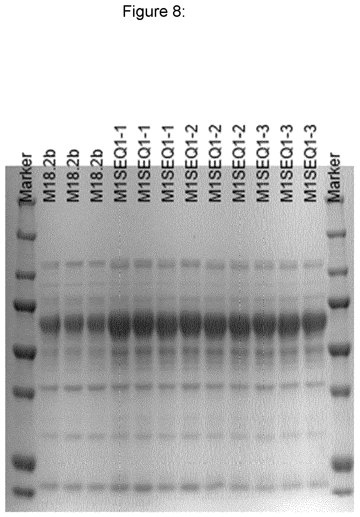

The viscosity of the culture broths of strains M1SEQ1-1 to -3 and M18.2b was measured using a Malvern Kinexus Lab+ KNX2110 rotational rheometer with the Vane tool (4Vnn:CUPnn) according to the manufacturer's instructions. The measurements were taken at a temperature of 20 °C and at a rotation velocity of 18.11 RPM ("rotations per minute"). The viscosity is depicted in Figure 7 and is presented in relation to the viscosity of the culture broth of strain M18.2b, which is set to 1. It is obvious from these data that the viscosity of the culture broths produced with M1SEQ1-1 to -3 is significantly lower than that of the host strain M18.2b. SDS-PAGE analysis of the centrifuged culture supernatants of strains M1SEQ1-1 to - 3 and M18.2b was done using methods known to those skilled in the art (e.g. described by Jansohn et al. (Gentechnische Methoden, Elsevier, München)) and the Criterion XT system (BioRad). Equal volumes of culture supernatants were loaded in each lane. Precision Plus Protein^{™} All Blue Standards (BioRad) was used as protein size reference. The gel image is shown in Figure 8. A person skilled in the art will recognize that the protein pattern of the *SEQ1* mutation strains M1SEQ1-1 to -3 is indistinguishable from that of the host strain M18.2b.

### Summary

Taken together these data demonstrate that the mutation of the *SEQ1* gene by creation of an early stop codon results in a significantly more efficient protein production, with more protein and less biomass being formed. The analysis of the secreted proteins by SDS-PAGE shows that their composition doesn't change significantly, indicating a general increase in protein production. In addition, the viscosity of the culture broth is significantly reduced as well.

### Example 3: Mutation of the SEQ1 gene (frame shift)

### Construction of a SEQ1 mutation vector

SEQ ID NO: 3, containing the flanking regions that introduce a G after T874 (position according to SEQ ID NO: 1) into the *SEQ1* gene, and a LIC site for insertion of the marker gene was synthesized by Thermo Fisher Scientific and cloned into a pUC19-derived plasmid. The resulting plasmid was named pSEQ1M2

Plasmid pSEQ1M2 was digested with *Srf*I (New England Biolabs) according to the manufacturer's instructions and purified using the Wizard PCR purification kit from Promega.

The hygromycin B resistance cassette (HygR) (SEQ ID NO: 4) was synthesized by Thermo Scientific. HygR was amplified by PCR using the DNA from Thermo Scientific as template, primers SEQ1MHygRfw (5'- AACAAGACACAGCCCTATAAC -3') and SEQ1MHygRrv (5'- AACAGACAAGAGCCCTATAAC -3') and phusion polymerase from Thermo Fisher Scientific according to the manufacturer's instructions (annealing temperature: 68.5 °C, elongation time: 40 sec, 30 cycles). The amplicon (2.4 kb) was purified using the Wizard PCR purification kit from Promega.

The PCR-amplified HygR marker was fused with linearized pSEQ1M1 using ligation independent cloning (LIC). The linearized vector was treated with T4 DNA polymerase in the presence of dTTP. The amplified promotors were treated with T4 DNA polymerase in the presence of dATP. T4 DNA polymerase treated vector and promotors were mixed and annealed as described in Ref. The assays were then transformed in chemically competent *Escherichia coli* XL1-Blue cells (Agilent), plated on LB-Agar plates containing 100 mg·l⁻¹ ampicillin (LB-Amp) and incubated at 37 °C for 24 h. Colonies were picked from the agar plates using toothpicks, transferred into liquid LB-Amp medium and incubated at 37 °C for 24 h with shaking (250 RPM). Plasmid DNA was isolated and integration of the insert was verified by digestion with *Xmn*I*.* Plasmid clones were verified by Sanger sequencing and one plasmid with correct sequence was designated pSEQ1M2-HygR.

### Transformation of the SEQ1 mutation vector into Trichoderma reesei

Vector pSEQ1M2-HygR was digested with *Xmn*I (New England Biolabs) according to the manufacturer's instructions and the mutation cassette (6.4 kb) was purified by agarose gel electrophoresis and with the Wizard PCR purification kit from Promega. *Trichoderma reesei* M18.2b (DSM 19984) was transformed with the digested vector essentially as described in Penttilä et al (1987) Gene 61: 155-164 or Gruber et al (1990) Curr Genet 18: 71-76. The transformants were selected on potato dextrose agar plates containing 100 mg·l⁻¹ of hygromycin and 1 M sorbitol and purified by singularisation. Conidia stocks of the purified strains were prepared by growing them on potato dextrose agar plates at 30 °C until the plates were covered with spores. The conidia were harvested with sterile sodium chloride (0.9 g·l⁻¹)-Triton X-100 (0.01 g·l⁻¹) solution, adjusted to OD₆₀₀ = 10, supplemented with 50 g·l⁻¹ of glycerol and stored at -80 °C.

Genomic DNA was isolated from the mycelium of the transformants and the host strain. The integration of the *SEQ1* mutation cassette at the intended locus was verified by PCR using phusion polymerase from Thermo Fisher Scientific according to the manufacturer's instructions, genomic DNA from the transformants as template and primers SEQ1M2KOfw (5'- GATGGCTGTGTAGAAGTAC -3') and SEQ1M2KOrv (5'- ATGAATAGGAGTGTGTGTG -3') (annealing temperature: 62.2 °C, elongation time: 1 min 25 sec, 30 cycles). A 2.5 kb band with primers SEQ1M2KOfw and SEQ1M2KOrv indicates the integration of the mutation cassette at the *SEQ1* locus. Genomic DNA from strain M18.2b was also tested as a control. In order to verify that the intended mutation had been inserted into the SEQ1 ORF, the respective region was amplified by PCR using phusion polymerase from Thermo Fisher Scientific according to the manufacturer's instructions, genomic DNA from the transformants as template and primers SEQ1M2Seqfw (5'- GACAGAAGCTCAAAGATTAG -3') and SEQ1M2Seqrv (5'- CAAGTCAAAGTCATGAGG -3') (annealing temperature: 63.6 °C, elongation time: 50 sec, 30 cycles). The 1.5 kb amplicon was purified using the Wizard PCR purification kit from Promega and sequenced using Primer M2Seq-01 (5'- CACTCTGTAAAGGCAAAGGG -3').

A strain containing the mutation from pSEQ1M2-HygR in the *SEQ1* ORF was named M2SEQ1-1.

### Growth of the SEQ1 mutation strain in shake flasks

The strains M2SEQ1-1 and M18.2b were grown in shake flasks in Hydrolyate Medium 1 as defined before for example 1. The medium was adjusted to pH 5.5 with HCl or NaOH and sterilized by autoclaving (20 min at 121 °C).

15 ml of the medium were distributed into 50 ml Erlenmeyer shake flasks under a sterile hood. Conidia stocks of strains M2SEQ1-1 and M18.2b were thawed, 75 µl of the conidia suspensions were pipetted into the Erlenmeyer flasks with the medium under a sterile hood and the flasks were closed with rubber foam caps. Three flasks were inoculated per strain. The flasks were incubated at 30 °C with shaking (250 RPM) for 6 days. After 6 days, the cultures were poured into 15 ml tubes. Aliquots were removed, centrifuged (3220xg, 4 °C, 15 min) and the supernatants stored at 4 °C, while the remaining culture broth was used for determination of the biomass and viscosity (see below).

### Characterization of the culture supernatants and broths: Protein concentration, SDS-PAGE, Biomass, Viscosity

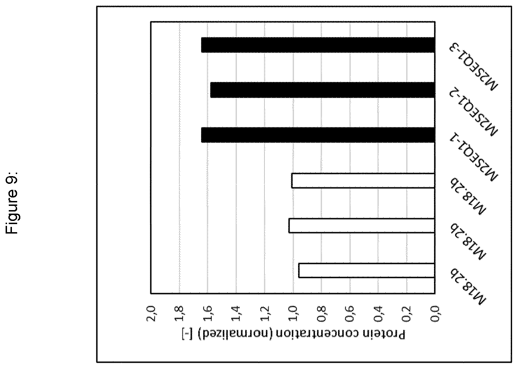

Protein concentrations in the centrifuged culture supernatants of strains M2SEQ1-1 and M18.2b were measured using the Quick Start^{™} Bradford reagent (BioRad) and BSA standard solutions (BioRad) according to the supplier's instructions. The results of the measurements are shown in Figure 9. It is obvious from these data that strain M2SEQ1-1 produce significantly more protein than the host strain M18.2b.

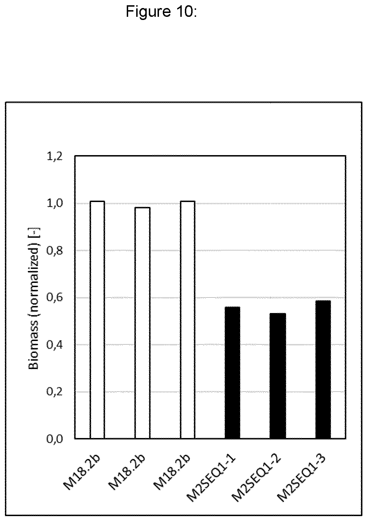

For biomass determination, Whatman^{™} filter discs (P1) were dried at 60 °C until their weight remained constant for 24 h. Culture broths of strains M2SEQ1-1 and M18.2b were filtered using those dried filter discs and the mycelia were washed with at least ten times the broth's volume of deionized water. Then the filter discs with the mycelium were dried at 60 °C until their weight remained constant for 24 h. The filter discs with the dried mycelia were weighed. The biomass concentration in the culture broth was then calculated by subtracting the mass of the dried filter disc from the mass of the dried filter disc with the mycelia and then dividing that value by the volume of the culture broth that had been filtered. The results of the measurements are shown in Figure 10. It is obvious from these data that strain M2SEQ1-1 produce significantly less biomass than the host strain M18.2b.

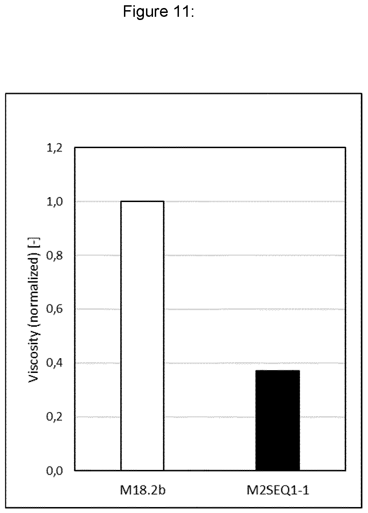

The viscosity of the culture broths of strains M2SEQ1-1 and M18.2b was measured using a Malvern Kinexus Lab+ KNX2110 rotational rheometer with the Vane tool (4Vnn:CUPnn) according to the manufacturer's instructions. The measurements were taken at a temperature of 20 °C and at a rotation velocity of 18.11 RPM ("rotations per minute"). The viscosity is depicted in Figure 11 and is presented in relation to the viscosity of the culture broth of strain M18.2b, which is set to 1. It is obvious from these data that the viscosity of the culture broths produced with M2SEQ1-1 is significantly lower than that of the host strain M18.2b.

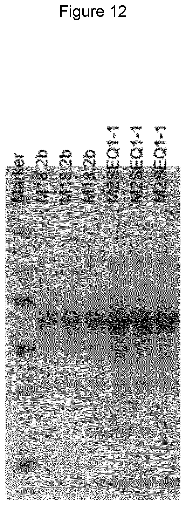

SDS-PAGE analysis of the centrifuged culture supernatants of strains M2SEQ1-1 and M18.2b was done using methods known to those skilled in the art (e.g. described by Jansohn et al. (Gentechnische Methoden, Elsevier, München)) and the Criterion XT system (BioRad). Equal volumes of culture supernatants were loaded in each lane. Precision Plus Protein^{™} All Blue Standards (BioRad) was used as protein size reference. The gel image is shown in Figure 12. A person skilled in the art will recognize that the protein pattern of the *SEQ1* mutation strain M2SEQ1-1 is indistinguishable from that of the host strain M18.2b.

### Summary

Taken together these data demonstrate that the mutation of the SEQ1 gene by creation of a frame shift results in a significantly more efficient protein production, with more protein and less biomass being formed. The analysis of the secreted proteins by SDS-PAGE shows that their composition doesn't change significantly, indicating a general increase in protein production. In addition, the viscosity of the culture broth is significantly reduced as well.

### Sequence listing

SEQ ID NO: 1
   SEQ1 native gene 
ATGAGGGCCTATCAGATCGAGATGCTCGACAAGAGCCTCAAGCAAAATGTCATT
GTTGCTGTATGTTGAAGTTTCTCTCCAATCCCCCGTCTCCCCCTTTGCTGTCGTT
GTCTTCGACGTTGAAAGACATGTCCATTGACCAAGGGGCGTTGTTATAAATCTA
GATGGACACGGGAAGTGGCAAGACTCAAGTGTAAGTTGTGCATCTTCATCATCG
GCAGCCCACGTAACCTGTGCCAGCCCTTAGCACCCTTCTTCGCAAAAGACTGAC
TTGGCGCTTGCATCAGAGCTGTGCTTCGTATCAAGAAGGAGCTGGAAATCTGCG
ATGCATCAAAGGTGAGTCTGCCGTCTGGATACAGTTGCACAACGACCTGGACAG
CTGCACTGACGCAGCACGCATCAGATCATCTGGTTCATCGCGCCAACAGTTTCG
CTGTGTCATCAGCAACACGATGTGCTCAAGTTGCAGATACCTGCCGTGCCCATG
ATGACACTGGCCGGGAACTCCAATATCGATGCTTGGGGGCCGGATATCTGGGC
CATTCTTCTCGACACGGTTCGAATTGTCATATCCACACCCCAGGTTCTGCTCGAT
GCCCTTGACCATGCTTACCTGAACTTGGGTCTTCTGGCGCTGCTTGTATTTGAT
GAAGGTATGGGACGACCTGCCTTCACTCTGTAAAGGCAAAGGGGCCGCCAGAA
GTTGCAAATCGCTGACGTGTCTTGTGCAAAAGTCCACAACTGCATTGGCAGAAG
TCCAGGCGGCAAAATCATGCTCCACCACTACCATCCGCGCAAGCTGGCTGGTG
AAAGCGTGCCTGCTGTTCTGGGTCTGACGGCAACTCCGAGCATTCAGTCTGAG
CTTGCCGATATTGATGCCTTGGAATGGCTGATGGATGCAAGATGCGTCTCGCCC
ACTCTCCATCGCGACGAACTGCTCAAATGCGTCAAGAGGCCCAATATCAAGCAC
ATCATCTATAAAGCCGGCAAAGAAGACATCACGACGCCCACCATGCGCGACTTG
GATCGGGTCTACCGGGCGCTGGACATTCTCGAAGACCCCTACATACTCATGCT
GCGCAACAACCCTACGGACCGAAACAACCGCCTGCTGCTAACAGCCATTGAAA
AGTACGATACCTACACACAGAACCAGATGAAGTCGTTCTGCGCCCGATCAAGAG
AGATATGCAAGCAACTCGGTCCCTGGGCTGCTGACCTCTTCATCTGGAAGGCCA
TCTCAGCTCACTTGGACAAGGTGGACAGGCAGACGGATGGAGTTGACGAGTAT
 
GGCAACAAGTGGTCGTCGGGGTCGACAAGCTTCCTGGAAAAGAAGCACCTGGC
CGACATCTATCGTCGAGTCAAGGTCCAACGTCCTTCCGATGTGCCACAGGTCTT
TGAAGACATTTCCGACAAGGTCGGTAAGCTAATCTTTGAGCTTCTGTCGGTAGA
GGAGCCCACGGTGGGCATCATCTTCGTCGAGGAACGAGTCATGGTTGCTATGC
TGGCCGAGGTTCTCTCTGTCAACCACACAATCACGTCCCGGTACCGGATCGGG
ACCATGGTTGGCACCTCAAATTACGCTGGGCGGCGGAAGGCCGTTTATGACTT
CGACCAGAAAACGGACTACAAGGACCTGCAGAGCTTCCGCTCCGGCAAGATTA
ACCTGCTGATTGCGACGTCAGTGCTGGAGGAGGGCATCGACGTGCCTGCCTGC
AACCTAGTCATATGCTTTGACACTCCGACGACCCCAAAGTCCTTTATCCAGCGG
CGCGGACGGGCTCGCTCCAAGGACTCGAATCTCCTTCTTTTCTTTGACGATGCC
AACCCTGCGATCTTGAAGTGGCAGGCGAAAGAGGAGGAGATGAACAGGATCTT
CGAAGACGAAGAGAGGGCGATTCGCGAACTCGGCAAACTGGAAGATTCGGAGA
GTCCGAGCACCATCTCCTTCACCGTCCCGTCTACCGGCGCAAGGCTAGATTTTG
ACAATGCGAAGCAGCACCTCGAGCACTTCTGCAGAGTCTTGTGCCCGTCGGAC
TTTGTGGACAGCCGCCCGGACTACATCATCCGCAGGGAGCAGGACTCTCCTTT
GTTGACTGCCATTGTACTGCTCCCTCCGTTTCTGCCGGTGAATCTGAGGCAGCA
CACCAGTGCTTCTCCTTGGCGCTCCGAGAAGAACGCCACCAAGGATGCTGCGT
ATCAGGCGTATATAGCCCTGTATGACGCGAAGCTCGTCAACGAGAACCTGCTGC
CCTTCAAGTCCAGCGACATGCTCGGAATCGATAAGCGAGTATCCGAGGTGCCG
GTCGAGCCGTTGATGAAGCCATGGCATCGTGTCGCTCCTGCGTGGCGGGAAGC
TGGCGACAAGTGGCTTTACTCCTTGAGCTGCGTGGAGGAGGACGGCCGAGTAA
GTGCAGAGTACGAGGTTCTGCTGCCAGTCTGGCTGAACCAGCCTCAGCCCCTG
AAAATGTTCCTCGACCGCAATCACCAGGTGGAGTTGCAGCTGAAGGCCGGGAT
ACCCGTGCCGCACGAGCAAGTTGCGTCCCTGCCAGATCATACATCGACTTTGCT
GGCGCTGCATTTCGGTCATCGATGGCCTCTCGAGCAGAAAGAGCACGTCATTC
GGGTCTGGGCCAAGGATCAACCCCTATCGCTGAACCAAATTGGCGAGCTCACA
TACGATCCACAGAATGAGAGCGTCAGCCGGGGAGAGTTTCTCATCCGGGACAA
CACCAGAGCCCCCTACCTGTACAAGGATACCATTGCGTTCAAGCCCGAACCGA
GCCAGGTCCAGAATACCTTTTACGAGTACGACAAGGCGCCCGAAGACGTGCCG
TATCTCGTGCTCACCAAATGGACGCGGCGGACCGACTTTCTGCATCGCCTCCAA
GGGAATCCCGCCAAGAATGAGGTTAGTAGCAAGCCATACGCACGCGTATATCC
GCTGTCGTGGGCGACAGTCGATACCATCCCCGCCAGGCACGCCCAGTTTGGCA
TGCTGATCCCGACCATGATCCACGAGCTCGGCGTCATGCTCATGGCCAAGGAG
CTGGCCTACTCCGTTCTCGACGAGGTTGGCATTTCGGATCTGCAGCTGGTCAAG
 
GAGGCCATCAGCGCGCGGAGTGCCTCGGAGCCGGTGAATTACGAGAGGCTGG
AGTTTTTGGGCGACTCGATTCTCAAGTTTTGTGCCTGTATGCGCGCCGCTGCTG
AAAGTAAGTTGCTCAAGCGTTTTACTCATATATGACTCCTGTGTGCACCTGTCCT
CTGACATGGAACTGTTTTGCTGACCACATTTGATACTGCCTAGAACCCGACTATC
CCGAGGGCTATCTCTCGTATTGGAGAGACCGACTCGTCTCCAACTCGAGGCTG
TACAAAGCCGCTCTCGAGTTTGGGCTGCCGAGGTTCATCTTGACGAAACCTTTT
ACCGGTCAAAAGTGGCGCCCACTCTACCTGGACGAGGTCCTCCAGCAAGGGGA
CGTCGCTACGCCGGAGAAGAGAAAATTATCGACCAAGACGCTCGCAGACGTGG
TCGAGGCGCTGATCGGGGCCTCATACGTCGATGGAGGCCTTTCAAAGGCAGTG
ACTTGCATCTCAAAATTCGTCCCCGAAGGCTCGTGGACCAGTGTTGATGCAGAT
AGAGAGTCTCTCTTTGCGAGAGTGCCAGACGGCGAGCCTCTCCCGCCGCCATT
GGAGCCGCTGGAGAAGTTGATCGGCTACACGTTCCAGAAAAAGGCGCTCTTGA
TGGAGGCTCTGACGCATGCCTCGTATGCTGCAGACTTCGGAACGCGATCTCTC
GAGAGGCTCGAATTCATAGGAGACGCTGTCCTGGACAACATTATCGTTACGAAG
CTCTTTAGGCTGAAGCCAGCGCTGCCCCATTTCAGGATGCATACGCTGAAGACG
GGCCTGGTGAATGGGGACTTTCTTGCTTTCATGACAATGGAGCACGGAGTGCAA
CTGGCGGCGGACCCTGTGGTGACAGAAGAAGCTACGGTGGAGGTCCCGGAAA
CGATTTCCTACCTGTGGTCGTTTTTGAGGCAGGCCTCTTTTCCCATTGCCATCGA
GCTGAAGGAGACGAACAAGCGGCACGCTGCCCTGAGAGAGCAGATTCACGAAG
CAATGGACAATGACGATCATTACCCCTGGGCGCTGCTGGCCGCCCTGAGCCCG
AAGAAGTTCTACTCTGACCTCTTCGAGGCGGTTCTCGGCGCTGTGTGGATCGAC
TCCGGGTCGCTGGCGGCGTGCGAGGGCATGGTTGCGCAGTTTGGGATCTTAAA
GTACATGGATCGGCTGCTGCGTGACGAAGTCCACGTGCAGCATCCTAAGGAGG
AGCTGGGCATGTGGGCAAACACAGAGACTGTGACGTACGAGCTCGAGATGAAG
GGGAGCGAGGAGAGCGCGGGGGAGAGGGAGTATTTCTGCAAGGTGTTTGTTG
GAAAGAGGGAGGTTGTGGAGGTTCGTGGGGGGGTCAATAAGGAGGAGGTGAA
GACGAAGGGTGCGACGGAGGCGTTGCGGATTTTGAGGGAGGAGAAAAGGCGC
GGTGCTGAGGATGTGGTGATGGTGGGATAA
SEQ ID NO: 2
   SEQ1 mutation flanks 1 
GACTGAAGGCCTTCATCAAATACAAGCAGCGCCAGAAGACCCAAGTTCAGGTAA
GCATGGTCAAGGGCATCGAGCAGAACCTGGGGTGTGGATATGACAATTCGAAC
CGTGTCGAGAAGAATGGCCCAGATATCCGGCCCCCAAGCATCGATATTGGAGT
 
TCCCGGCCAGTGTCATCATGGGCACGGCAGGTATCTGCAACTTGAGCACATCG
TGTTGCTGATGACACAGCGAAACTGTTGGCGCGATGAACCAGATGATCTGATGC
GTGCTGCGTCAGTGCAGCTGTCCAGGTCGTTGTGCAACTGTATCCAGACGGCA
GACTCACCTTTGATGCATCGCAGATTTCCAGCTCCTTCTTGATACGAAGCACAG
CTCTGATGCAAGCGCCAAGTCAGTCTTTTGCGAAGAAGGGTGCTAAGGGCTGG
CACAGGTTACGTGGGCTGCCGATGATGAAGATGCACAACTTACACTTGAGTCTT
GCCACTTCACGTGTCCATCTAGATTTATAACAACGCCCCTTGGTCAATGGACAT
GTCTTTCAACGTCGAAGACAACGACAGCAAAGGGGGAGACGGGGGATTGGAGA
GAAACTTCAACATACAGCAACAATGACATTTTGCTTGAGGCTCTTGTCGAGCATC
TCGATCTGATAGGCCCTCATGACTTTGACTTGCTCTCCCTCCTCGCCCAGCAGT
GCTGCCTGGGAGCCTGAGGTTGTTGTCGGGATGGCTCCATCTTGACCGGGCGT
AGGCTGCTTGATATCAGCTCCAGGCACGGCAGCCTCGTCGGCGACATGGCCAT
CGGGAGCACCATCCGACCCCGTGGCCGCGCCCGCCCCTTTCTCGACGGCGTG
GCCAGTCACATAGTTGAGCGATGGAGCCACGTCTCCAGAGGAGCAAGACGACC
ATGAATCCGCGTCCGACGACGACGACGAGCGTGGGCGCACCCGCGGCACGTC
ATCCCCAGCGCTCGCCAGGCCCGGAGCAGCAGCCGAAGCAGCGCGGACAGGA
CCAGGACCAGACCCAGGCCCTGACCCTGACCCTGATGGCAGCCCAGGGCCAG
CCGCCGCTACTAGCTGCAACATGGAAAGGCGACGGCGGCAGTTTGGCGCTCCC
GCGGCGGCGGATCCCGGGGTGTGACTCAGGCAGCGGCCCCTGCGAAGCACCC
GCAGCCTCAGGGAGCGTATTCTGACGTGTCGGGCAGACGCAACGAGTGCGTAT
CGAGCGACTAGCTGCGCGTGAATCCCGGCTCGCGATGCCTCACGGCGACGGC
GGCGGAGTTGGTTGCGGGGTTGGCGACTCGACGCTGGCGGGCTGGGACGCG
GATTTGCACCTGGATCACCTGGATCTGGAGCTGGAGCTGCTGGATTCATGCCC
CTGCTCGGCGGGAAGCCGGTGATTTGCGTGGCTGCCACTGGGGAGCTAAGAG
AGAGGCAAGCCGTAGTCTTAGTCGTAGTTATATGTAGTTGTAAGGTAAGGCAAG
GTACATGTAGTCGTCTGTAGGGCCTGTCTAGCGAACCTTGAGGTTTCCTGGATC
CACGGCGAGCTTGGCCAGCATCAGAGAGAGGAGGAGGCAGAGAGGCGTC
AACAGCTGAGGCGTCACGGCTCGAGTGTCACAGGCATCGCTTCCCCGCAGGTG
GAACAAGGCGTGTGTCCTTTCGTTGGGCTGCTGAGATGCCAATTGCTTGCTTGC
TGGGGGTGAGAGAGAGGCCGCAGAGGTGTAGGAGGAGGAGACGCAATTGGGT
GACGTGGGGGGAGTGAATGAATACAACGTCAAAGCAGGACTGCGAATTCAATC
TATGGCAGGCAAGGACACTCGTGCACGACAGGCTACATAGCTACTATTAGCAAA
CGGAGGATGTTGTTTTTGTGTTGGTTCATGCAGCCACTCATGCAGAGCCCCGGC
CTCTTCTCACCTTGTTTGGGTTGTTGAAGCTTGTTTCGTGGCTTGATAGTGAGCC
 
GCTGCGTTAAGATTCAAACTGTCACCGTTAGCGTCGGCAGCCTCGTTCACGGCC
TCTCCTTGGTTTCTGGAACTCTCACAGGTATGTCAAGCAAGAATGTAACAAGACA
CAGCCCGGGCTCTTGTCTGTTACCAGGTCATATGAGCCAGGGATCAAGAAATCC
GCGGAAAGGTCAATAAAATGGGAGTTACTGAGTATATTCCGTCCCGTGCCTTTT
CTTCATGCTGGTCTCTGGAGGAAATCTCACGGCTATCGTCACGAATGGCCGAAT
AAGTGCGACCATCTTCAGAGCTTCGTGTGACTGGCGACCATCTTTACCCGAAGT
CAGATAGACAGGAATCACCATTGGTAGCAGTGACTAATTAATGCACTGTTACAAC
ACATGACTGACAAGTTTCGAAAACCAAGAATAAGGTACTATCAATATCATAAGTA
CCTAGGATCTAAGGTATGTAGGCAGACCGTCATCATCACCTTCAAACCCCCGAC
AGGACAGGCCTCAATCCCCGGCAGTAGGTACATACTCTATCTCCGGGACTTGAC
GACCCACCATTGCGATTGCCGCCTCAATGCCAGAAGGAACTTTTGTCCTCCCAG
TGCTTTGTCCTGCTACTGTCATTACCAAGCAAGCCTTAGGAGTCATAAGAGTCAT
ACTGAACCTTAGGTACTTGTTGGGCAGAGCCATGTGGCCTACACTCCCAAACTC
AAAATTACTGGTCCGTTCATGCCTTGGATCATGTATCTTTCCATTTGCCAATCATA
GGCCTCCCGGAACTTTCAAGCAATAGAGAGGTCAGCTCGATGCTGGACAGGGC
AGACCGTACTTACACGTACCTAGGTAGCCTCTCTGTAGAACCTTTGACCCTCAA
AAGGTTCATCACCAAGTTATTGCGTAGCATGACCTAGAATACTGAAATTAGATCG
CGCAAGTAGCAATAATTCGCTATACTATGTATGCGGCAAGTCGCATTTCAGAAG
CCGGTTCTGTTCAACCACAGTCCAACCGTCGTCAATCTGGAGATGCGTCACAGG
AGCCGCAGGTGACTGCACAGCGATGCACCGTGGTGATGACATTGAATCTGCCT
AGGTATAATTACCTACATTTTCAATGTCTGTCTTCAAAAATAGAATAAAGTGCCAC
TTGGCATTCGACAATTGTTGTGTTGAACAATTGGGAGTATCTACGTCGTGAATCA
TGTTGCAAGCAAGAGGTATAGGTAGGTACCTTACCTGTTGAAGCAGCTAGCGCC
CTAGAGGCAGCCTCTGATGTCGTCTTGTCATTTTTTGATCCATCTCAAACAAGTC
TCAACAACGCATCCCATGCACCATGGAGCTTTTCGCAACAGGCTTCAACGCCTG
GAACCAGCTCACTTTCACCAAAGGCAGCCCCCAAGAGGCCATCCCAGAGGAAC
CAGATGACCTCTTCGGCTTTACAAAAGTTCTCTCCGCCACATCCATTGAACGGC
CAGTATCGCGCCTCACTTACACCATCGGTACCTTATATCAAATCACAACCTATCT
CATACCCCATCACACCTCCCTTTTACGCCCTATAAAAGACCATTACCGCCTAAAC
CTCAACAATCAAACCACAAACTGACAAAAGATTTCCCCCCCCTCCAGTCCGAAA
AGACGACCACCTCATCCTCGCCGGCCATGGCCCCTCGCAGCACAACCTCGAGC
ACCTCTACGCGTCCGCCGAAACGTTTCGACT
SEQ ID NO: 3
   SEQ1 mutation flanks 2 
GACTGAAGTACTTCATCTGGTTCTGTGTGTAGGTATCGTACTTTTCAATGGCTGT
TAGCAGCAGGCGGTTGTTTCGGTCCGTAGGGTTGTTGCGCAGCATGAGTATGT
AGGGGTCTTCGAGAATGTCCAGCGCCCGGTAGACCCGATCCAAGTCGCGCATG
GTGGGCGTCGTGATGTCTTCTTTGCCGGCTTTATAGATGATGTGCTTGATATTG
GGCCTCTTGACGCATTTGAGCAGTTCGTCGCGATGGAGAGTGGGCGAGACGCA
TCTTGCATCCATCAGCCATTCCAAGGCATCCAATATCGGCAAGCTCAGACTGAA
TGCTCGGAGTTGCCGTCAGACCCAGAACAGCAGGCACGCTTTCACCAGCCAGC
TTGCGCGGATGGTAGTGGTGGAGCATGATTTTGCCGCCTGGACTTCTGCCAAT
GCAGTTGTGGACTTTTGCACAAGACACGTCAGCGATTTGCAACTTCTGGCGGCC
CCTTTGCCTTTACAGAGTGAAGGCAGGTCGTCCCATACCTTCATCAAATACAAG
CAGCGCCAGAAGACCCAAGTTCAGGTAAGCATGGTCAAGGGCATCGAGCAGAA
CCTGGGGTGTGGATATGACAATTCGAACCGTGTCGAGAAGAATGGCCCAGATAT
CCGGCCCCCAAGCATCGATATTGGAGTTCCCGGCCAGTGTCATCATGGGCACG
GCAGGTATCTGCAACTTGAGCACATCGTGTTGCTGATGACACAGCGAAACTGTT
GGCGCGATGAACCAGATGATCTGATGCGTGCTGCGTCAGTGCAGCTGTCCAGG
TCGTTGTGCAACTGTATCCAGACGGCAGACTCACCTTTGATGCATCGCAGATTT
CCAGCTCCTTCTTGATACGAAGCACAGCTCTGATGCAAGCGCCAAGTCAGTCTT
TTGCGAAGAAGGGTGCTAAGGGCTGGCACAGGTTACGTGGGCTGCCGATGATG
AAGATGCACAACTTACACTTGAGTCTTGCCACTTCCCGTGTCCATCTAGATTTAT
AACAACGCCCCTTGGTCAATGGACATGTCTTTCAACGTCGAAGACAACGACAGC
AAAGGGGGAGACGGGGGATTGGAGAGAAACTTCAACATACAGCAACAATGACA
TTTTGCTTGAGGCTCTTGTCGAGCATCTCGATCTGATAGGCCCTCATGACTTTGA
CTTGCTCTCCCTCCTCGCCCAGCAGTGCTGCCTGGGAGCCTGAGGTTGTTGTC
GGGATGGCTCCATCTTGACCGGGCGTAGGCTGCTTGATATCAGCTCCAGGCAC
GGCAGCCTCGTCGGCGACATGGCCATCGGGAGCACCATCCGACCCCGTGGCC
GCGCCCGCCCCTTTCTCGACGGCGTGGCCAGTCACATAGTTGAGCGATGGAGC
CACGTCTCCAGAGGAGCAAGACGACCATGAATCCGCGTCCGACGACGACGACG
AGCGTGGGCGCACCCGCGGCACGTCATCCCCAGCGCTCGCCAGGCCCGGAGC
AGCAGCCGAAGCAGCGCGGACAGGACCAGGACCAGACCCAGGCCCTGACCCT
GACCCTGATGGCAGCCCAGGGCCAGCCGCCGCTACTAGCTGCAACATGGAAAG
GCGACGGCGGCAGTTTGGCGCTCCCGCGGCGGCGGATCCCGGGGTGTGACTC
AGGCAGCGGCCCCTGCGAAGCACCCGCAGCCTCAGGGAGCGTATTCTGACGT
GTCGGGCAGACGCAACGAGTGCGTATCGAGCGACTAGCTGCGCGTGAATCCC
 
GGCTCGCGATGCCTCACGGCGACGGCGGCGGAGTTGGTTGCGGGGTTGGCGA
CTCGACGCTGGCGGGCTGGGACGCGGATTTGCACCTGGATCACCTGGATCTGG
AGCTGGAGCTGCTGGATTCATGCCCCTGCTCGGCGGGAAGCCGGTGATTTGCG
TGGCTGCCACTGGGGAGCTAAGAGAGAGGCAAGCCGTAGTCTTAGTCGTAGTT
ATATGTAGTTGTAAGGTAAGGCAAGGTACATGTAGTCGTCTGTAGGGCCTGTCT
AGCGAACCTTGAGGTTTCCTGGATCCACGGCGAGCTTGGCCAGCATCAGAGAG
AGGAGGAGGAGGCAGAGAGGCGTCAACAGCTGAGGCGTCACGGCTCGAGTGT
CACAGGCATCGCTTCCCCGCAGGTGGAACAAGGCGTGTGTCCTTTCGTTGGGC
TGCTGAGATGCCAATTGCTTGCTTGCTGGGGGTGAGAGAGAGGCCGCAGAGGT
GTAGGAGGAGGAGACGCAATTGGGTGACGTGGGGGGAGTGAATGAATACAAC
GTCAAAGCAGGACTGCGAATTCAATCTATGGCAGGCAAGGACACTCGTGCACG
ACAGGCTACATAGCTACTATTAGCAAACGGAGGATGTTGTTTTTGTGTTGGTTCA
TGCAGCCACTCATGCAGAGCCCCGGCCTCTTCTCACCTTGTTTGGGTTGTTGAA
GCTTGTTTCGTGGCTTGATAGTGAGCCGCTGCGTTAAGATTCAAACTGTCACCG
TTAGCGTCGGCAGCCTCGTTCACGGCCTCTCCTTGGTTTCTGGAACTCTCACAG
GTATGTCAAGCAAGATAACAAGACACAGCCCGGGCTCTTGTCTGTTACCAGGTC
ATATGAGCCAGGGATCAAGAAATCCGCGGAAAGGTCAATAAAATGGGAGTTACT
GAGTATATTCCGTCCCGTGCCTTTTCTTCATGCTGGTCTCTGGAGGAAATCTCAC
GGCTATCGTCACGAATGGCCGAATAAGTGCGACCATCTTCAGAGCTTCGTGTGA
CTGGCGACCATCTTTACCCGAAGTCAGATAGACAGGAATCACCATTGGTAGCAG
TGACTAATTAATGCACTGTTACAACACATGACTGACAAGTTTCGAAAACCAAGAA
TAAGGTACTATCAATATCATAAGTACCTAGGATCTAAGGTATGTAGGCAGACCGT
CATCATCACCTTCAAACCCCCGACAGGACAGGCCTCAATCCCCGGCAGTAGGT
ACATACTCTATCTCCGGGACTTGACGACCCACCATTGCGATTGCCGCCTCAATG
CCAGAAGGAACTTTTGTCCTCCCAGTGCTTTGTCCTGCTACTGTCATTACCAAGC
AAGCCTTAGGAGTCATAAGAGTCATACTGAACCTTAGGTACTTGTTGGGCAGAG
CCATGTGGCCTACACTCCCAAACTCAAAATTACTGGTCCGTTCATGCCTTGGAT
CATGTATCTTTCCATTTGCCAATCATAGGCCTCCCGGAACTTTCAAGCAATAGAG
AGGTCAGCTCGATGCTGGACAGGGCAGACCGTACTTACACGTACCTAGGTAGC
CTCTCTGTAGAACCTTTGACCCTCAAAAGGTTCATCACCAAGTTATTGCGTAGCA
TGACCTAGAATACTGAAATTAGATCGCGCAAGTAGCAATAATTCGCTATACTATG
TATGCGGCAAGTCGCATTTCAGAAGCCGGTTCTGTTCAACCACAGTCCAACCGT
CGTCAATCTGGAGATGCGTCACAGGAGCCGCAGGTGACTGCACAGCGATGCAC
CGTGGTGATGACATTGAATCTGCCTAGGTATAATTACCTACATTTTCAATGTCTG
 
TCTTCAAAAATAGAATAAAGTGCCACTTGGCATTCGACAATTGTTGTGTTGAACA
ATTGGGAGTATCTACGTCGTGAATCATGTTGCAAGCAAGAGGTATAGGTAGGTA
CCTTACCTGTTGAAGCAGCTAGCGCCCTAGAGGCAGCCTCTGATGTCGTCTTGT
CATTTTTTGATCCATCTCAAACAAGTCTCAACAACGCATCCCATGCACCATGGAG
CTTTTCGCAACAGGCTTCAACGCCTGGAACCAGCTCACTTTCACCAAAGGCAGC
CCCCAAGAGGCCATCCCAGAGGAACCAGATGACCTCTTCGGCTTTACAAAAGTT
CTCTCCGCCACATCCATTGAACGGCCAGTATCGCGCCTCACTTACACCATCGGT
ACCTTATATCAAATCACAACCTATCTCATACCCCATCACACCTCCCTTTTACGCC
CTATAAAAGACCATTACCGCCTAAACCTCAACAATCAAACCACAAACTGACAAAA
GATTTCCCCCCCCTCCAGTCCGAAAATTTTCGACT
SEQ ID NO: 4
   Hygromycin B resistance marker

GTTAACAAGACACAGCCCTATAACTTCGTATAATGTATGCTATACGAAGTTATAT
AACGGTGAGACTAGCGGCCGGTCCCCTTATCCCAGCTGTTCCACGTTGGCCTG
CCCCTCAGTTAGCGCTCAACTCAATGCCCCTCACTGGCGAGGCGAGGGCAAGG
ATGGAGGGGCAGCATCGCCTGAGTTGGAGCAAAGCGGCCCGGCCGCCATGGG
AGCAGCGAACCAACGGAGGGATGCCGTGCTTTGTCGTGGCTGCTGTGGCCAAT
CCGGGCCCTTGGTTGGCTCACAGAGCGTTGCTGTGAGACCATGAGCTATTATTG
CTAGGTACAGTATAGAGAGAGGAGAGAGAGAGAGAGAGAGAGAGAGGGGAAAA
AAGGTGAGGTTGAAGTGAGAAAAAAAAAAAAAAAAAAAAATCCAACCACTGACG
GCTGCCGGCTCTGCCACCCCCCTCCCTCCACCCCAGACCACCTGCACACTCAG
CGCGCAGCATCACCTAATCTTGGCTCGCCTTCCCGCAGCTCAGGTTGTTTTTTT
TTTCTCTCTCCCTCGTCGAAGCCGCCCTTGTTCCCTTATTTATTTCCCTCTCCAT
CCTTGTCTGCCTTTGGTCCATCTGCCCCTTTGTCTGCATCTCTTTTGCACGCATC
GCCTTATCGTCGTCTCTTTTTTCACTCACGGGAGCTTGACGAAGACCTGACTCG
TGAGCCTCACCTGCTGATTTCTCTCCCCCCCTCCCGACCGGCTTGACTTTTGTT
TCTCCTCCAGTACCTTATCGCGAAGCCGGAAGAACCTCTTAACCTCTAGATGAA
AAAGCCTGAACTCACCGCCACGTCTGTCGAGAAGTTCCTGATCGAAAAGTTCGA
CAGCGTCTCCGACCTGATGCAGCTCTCGGAGGGCGAAGAATCTCGTGCTTTCA
GCTTCGATGTAGGAGGGCGTGGATATGTCCTGCGGGTAAATAGCTGCGCCGAT
GGTTTCTACAAAGATCGTTATGTTTATCGGCACTTTGCATCGGCCGCGCTCCCG
ATTCCGGAAGTGCTTGACATTGGGGAATTCAGCGAGAGCCTGACCTATTGCATC
TCCCGCCGTGCACAGGGTGTCACGTTGCAAGACCTGCCTGAAACCGAACTGCC

CGCTGTTCTGCAGCCGGTCGCGGAGGCCATGGATGCGATCGCTGCGGCCGAT
CTCAGCCAGACGAGCGGGTTCGGCCCATTCGGACCGCAAGGAATCGGTCAATA
CACTACATGGCGTGATTTCATATGCGCGATTGCTGATCCCCATGTGTATCACTG
GCAAACTGTGATGGACGACACCGTCAGTGCGTCCGTCGCGCAGGCTCTCGATG
AGCTGATGCTTTGGGCCGAGGACTGCCCCGAAGTCCGGCACCTCGTGCACGC
GGATTTCGGCTCCAACAATGTCCTGACGGACAATGGCCGCATAACAGCGGTCAT
TGACTGGAGCGAGGCGATGTTCGGGGATTCCCAATACGAGGTCGCCAACATCT
TCTTCTGGAGGCCGTGGTTGGCTTGTATGGAGCAGCAGACGCGCTACTTCGAG
CGGAGGCACCCGGAGCTTGCAGGATCGCCGCGGCTCCGGGCGTATATGCTCC
GCATTGGTCTTGACCAACTCTATCAGAGCTTGGTTGACGGCAATTTCGATGATG
CAGCTTGGGCGCAGGGTCGATGCGACGCAATCGTCCGATCCGGAGCCGGGAC
TGTCGGGCGTACACAAATCGCCCGCAGAAGCGCGGCCGTCTGGACCGATGGC
TGTGTAGAAGTACTCGCCGATAGTGGAAACCGACGCCCCAGCACTCGTCCGAG
GGCAAAGGAATAGATGCATGGCTTTCGTGACCGGGCTTCAAACAATGATGTGCG
ATGGTGTGGTTCCCGGTTGGCGGAGTCTTTGTCTACTTTGGTTGTCTGTCGCAG
GTCGGTAGACCGCAAATGAGCAACTGATGGATTGTTGCCAGCGATACTATAATT
CACATGGATGGTCTTTGTCGATCAGTAGCTAGTGAGAGAGAGAGAACATCTATC
CACAATGTCGAGTGTCTATTAGACATACTCCGAGAATAAAGTCAACTGTGTCTGT
GATCTAAAGATCGATTCGGCAGTCGAGTAGCGTATAACAACTCCGAGTACCAGC
GAAAGCACGTCGTGACAGGAGCAGGGCTTTGCCAACTGCGCAACCTTGCTTGA
ATGAGGATACACGGGGTGCAACATGGCTGTACTGATCCATCGCAACCAAAATTT
CTGTTTATAGATCAAGCTGGTAGATTCCAATTACTCCACCTCTTGCGCTTCTCCA
TGACATGTAAGTGCACGTGGAAACCATACCCAATATAACTTCGTATAATGTATGC
TATACGAAGTTATAGGGCTCTTGTCTGTTAAC

## Claims

1. Process for production of a whole broth enzyme composition, comprising the following steps:
(a) providing a fermentation medium, originating from hydrolysis of lignocellulosic biomass, with a glucose content of from 5 to 450 g/L, a xylose content of from 2 to 300 g/L, a density of from 1 to 2 kg/L and a dry matter content of from 10 to 75 wt.-%;
(b) addition of at least one filamentous fungus cell wherein SEQ1 gene sequence SEQ ID NO:1 has been disrupted;
(c) mixing of the fermentation medium and the at least one filamentous fungus cell for a time period of from 1 minute to 10 days at a temperature of from 20 to 35 °C;
(d) obtaining a whole broth enzyme composition.

2. Process according to claim 1, wherein the pH of the fermentation medium according to step (a) has been adjusted to a pH selected from pH 2.0 to 6.0.

3. Process according to any of the foregoing claims, wherein the ratio from glucose to xylose is from 1.0 to 3.5.

4. Process according to any of the foregoing claims, further comprising the step (ai) concentration of the fermentation medium by evaporation, membrane filtration or thin layer evaporation to decrease the weight of the fermentation medium by factor 2 to 6.

5. Process according to any of the foregoing claims, further comprising step (aii) sterilization of the fermentation medium according to step (a) or the concentrated fermentation medium according to step (ai).

6. Process according to any of the foregoing claims, wherein the fermentation medium according to step (a) has a furfural content of less than 0.5 g/L.

7. Process according to any of the foregoing claims, wherein the fermentation medium according to step (a) has a hydroxymethyl furfural (HMF) content of less than 0.5 g/L.

8. Process according to any of the foregoing claims further comprising the step (e) solid-liquid separation of the fermented medium according to step (c) to obtain a solid fraction and a liquid fraction.

9. Process according to any of the foregoing claims wherein from 0.05 to 5 wt.-% nitrogen are added during step (a) and/or (b) of the process.

10. Process according to any of the foregoing claims wherein from 0.5 to 350 mg/L FeSO₄, MnSO₄, MgSO₄ and/or ZnSO₄ are added during step (a) and/or (b) of the process.

11. Process according to any of the foregoing claims, wherein the filamentous fungus cell is *Trichoderma.*

12. Process according to any of the foregoing claims, wherein the filamentous fungus cell comprises at least one heterologous beta-glucosidase enzyme.

13. Filamentous fungus cell wherein SEQ1 gene sequence SEQ ID NO:1 has been disrupted

14. Filamentous fungus cell according to claim 13, wherein SEQ ID NO:1 has been disrupted by deletion, mutation, modification of a promotor or any other regulatory sequence, generation of a stop codon or RNA interference.

15. Filamentous fungus cell according to any of claims 13 or 14, wherein the at least one filamentous fungus cell is a genetically modified filamentous fungus cell with the ability to express at least one heterologous hydrolase enzyme, at least one heterologous pectinase enzyme, at least one heterologous oxidative enzyme and/or at least one heterologous accessory protein.

16. Filamentous fungus cell according to any of claims 13 to 15, wherein the at least one filamentous fungus cell is a genetically modified filamentous fungus cell comprising at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence.

17. Whole broth enzyme composition produced according to a process as defined in any of claims 1 to 12.

18. Use of a filamentous fungus cell as defined in any of claims 13 to 16 for the production of whole broth enzyme composition.

19. Use of a whole broth enzyme composition according to claim 18 for the hydrolyzation of lignocellulosic biomass.

## Patentansprüche

1. Verfahren zur Herstellung einer Ganzfermentationsbrühen-Enzymzusammensetzung, das die folgenden Schritte umfasst:
(a) Bereitstellen eines Fermentationsmediums, das aus der Hydrolyse von lignocellulosehaltiger Biomasse stammt, mit einem Glucosegehalt von 5 bis 450 g/L, einem Xylosegehalt von 2 bis 300 g/L, einer Dichte von 1 bis 2 kg/L und einem Trockensubstanzgehalt von 10 bis 75 Gew.-%;
(b) Zugabe von mindestens einer filamentösen Pilzzelle, in der die SEQ1-Gensequenz SEQ ID NO:1 disruptiert wurde;
(c) Mischen des Fermentationsmediums und der mindestens einen filamentösen Pilzzelle über einen Zeitraum von 1 Minute bis 10 Tagen bei einer Temperatur von 20 bis 35 °C;
(d) Gewinnung einer Gesamtfermentationsbrühen-Enzymzusammensetzung.

2. Verfahren nach Anspruch 1, wobei der pH-Wert des Fermentationsmediums gemäß Schritt (a) auf einen pH-Wert zwischen 2,0 und 6,0 eingestellt wurde.

3. Verfahren nach einem der vorangehenden Ansprüche, wobei das Verhältnis von Glucose zu Xylose 1,0 bis 3,5 beträgt.

4. Verfahren nach einem der vorangehenden Ansprüche, umfassend ferner den Schritt (ai) Konzentration des Fermentationsmediums durch Verdampfung, Membranfiltration oder Dünnschichtverdampfung, um das Gewicht des Fermentationsmediums um den Faktor 2 bis 6 zu verringern.

5. Verfahren nach einem der vorangehenden Ansprüche, das ferner den Schritt (aii) Sterilisation des Fermentationsmediums gemäß Schritt (a) oder des konzentrierten Fermentationsmediums gemäß Schritt (ai).

6. Verfahren nach einem der vorangehenden Ansprüche, wobei das Fermentationsmedium nach Schritt (a) einen Furfuralgehalt von weniger als 0,5 g/L aufweist.

7. Verfahren nach einem der vorangehenden Ansprüche, wobei das Fermentationsmedium nach Schritt (a) einen Gehalt an Hydroxymethylfurfural (HMF) von weniger als 0,5 g/L aufweist.

8. Verfahren nach einem der vorangehenden Ansprüche, das ferner den Schritt (e) Fest-Flüssig-Trennung des fermentierten Mediums gemäß Schritt (c), um eine feste Fraktion und eine flüssige Fraktion zu erhalten.

9. Verfahren nach einem der vorhergehenden Ansprüche, wobei 0,05 bis 5 Gew.-% Stickstoff während Schritt (a) und/oder (b) des Verfahrens zugesetzt werden.

10. Verfahren nach einem der vorangehenden Ansprüche, wobei 0,5 bis 350 mg/L FeSO₄, _{MnSO(4)} , MgSO₄ und/oder ZnSO₄ während Schritt (a) und/oder (b) des Verfahrens zugesetzt werden.

11. Verfahren nach einem der vorstehenden Ansprüche, wobei es sich bei der fadenförmigen Pilzzelle um *Trichoderma* handelt.

12. Verfahren nach einem der vorangehenden Ansprüche, wobei die Fadenpilzzelle mindestens ein heterologes beta-Glucosidase-Enzym enthält.

13. Filamentöse Pilzzelle, in der die SEQ1-Gensequenz SEQ ID NO:1 disruptiert wurde.

14. Filamentöse Pilzzelle nach Anspruch 13, wobei SEQ ID NO:1 durch Deletion, Mutation, Modifikation eines Promotors oder einer anderen regulatorischen Sequenz, Erzeugung eines Stoppcodons oder RNA-Interferenz unterbrochen wurde.

15. Fadenpilzzelle nach einem der Ansprüche 13 oder 14, wobei die mindestens eine Fadenpilzzelle eine genetisch veränderte Fadenpilzzelle mit der Fähigkeit ist, mindestens ein heterologes Hydrolase-Enzym, mindestens ein heterologes Pectinase-Enzym, mindestens ein heterologes oxidatives Enzym und/oder mindestens ein heterologes akzessorisches Protein zu exprimieren.

16. Filamentöse Pilzzelle nach einem der Ansprüche 13 bis 15, wobei die mindestens eine filamentöse Pilzzelle eine genetisch modifizierte filamentöse Pilzzelle ist, die mindestens eine heterologe Beta-Glucosidase-Enzymkodiersequenz, mindestens eine heterologe Beta-Xylosidase-Enzymkodiersequenz mindestens eine heterologe Xylanase-Enzymkodiersequenz, mindestens eine heterologe Pektinase-Enzymkodiersequenz, mindestens eine heterologe lytische Polysaccharid-Monooxygenase-Enzymkodiersequenz, mindestens eine heterologe oxidative Enzymkodiersequenz und/oder mindestens eine heterologe akzessorische Protein-Kodiersequenz.

17. Gesamtbrühen-Enzymzusammensetzung, hergestellt nach einem Verfahren gemäß einem der Ansprüche 1 bis 12.

18. Verwendung einer Fadenpilzzelle nach einem der Ansprüche 13 bis 16 zur Herstellung einer Gesamtbrühen- Enzymzusammensetzung.

19. Verwendung einer Gesamtbrühen-Enzymzusammensetzung nach Anspruch 18 für die Hydrolyse von lignozellulosehaltiger Biomasse.

## Revendications

1. Procédé de production d'une composition enzymatique de bouillon entier, comprenant les étapes suivantes :
(a) fournir un milieu de fermentation, provenant de l'hydrolyse de la biomasse lignocellulosique, avec une teneur en glucose de 5 à 450 g/L, une teneur en xylose de 2 à 300 g/L, une densité de 1 à 2 kg/L et une teneur en matière sèche de 10 à 75 % en poids ;
(b) ajout d'au moins une cellule de champignon filamenteux dans laquelle la séquence du gène SEQ1 (SEQ ID NO:1) a été perturbée ;
(c) mélange du milieu de fermentation et d'au moins une cellule de champignon filamenteux pendant une durée comprise entre 1 minute et 10 jours à une température comprise entre 20 et 35 °C ;
(d) l'obtention d'une composition enzymatique de bouillon entier.

2. Procédé selon la revendication 1, dans lequel le pH du milieu de fermentation selon l'étape (a) a été ajusté à un pH choisi entre 2,0 et 6,0.

3. Procédé selon l'une quelconque des revendications précédentes, dans lequel le rapport entre le glucose et le xylose est compris entre 1,0 et 3,5.

4. Procédé selon l'une quelconque des revendications précédentes, comprenant en outre l'étape (ai) concentration du milieu de fermentation par évaporation, filtration sur membrane ou évaporation en couche mince pour diminuer le poids du milieu de fermentation d'un facteur 2 à 6.

5. Procédé selon l'une quelconque des revendications précédentes, comprenant en outre l'étape
(aii) stérilisation du milieu de fermentation selon l'étape (a) ou du milieu de fermentation concentré selon l'étape (ai).

6. Procédé selon l'une quelconque des revendications précédentes, dans lequel le milieu de fermentation selon l'étape (a) a une teneur en furfural inférieure à 0,5 g/L.

7. Procédé selon l'une quelconque des revendications précédentes, dans lequel le milieu de fermentation selon l'étape (a) a une teneur en hydroxyméthyl furfural (HMF) inférieure à 0,5 g/L.

8. Procédé selon l'une quelconque des revendications précédentes comprenant en outre l'étape
(e) séparation solide-liquide du milieu fermenté selon l'étape (c) pour obtenir une fraction solide et une fraction liquide.

9. Procédé selon l'une quelconque des revendications précédentes, dans lequel de 0,05 à 5 % en poids d'azote sont ajoutés au cours de l'étape (a) et/ou (b) du procédé.

10. Procédé selon l'une quelconque des revendications précédentes dans lequel de 0,5 à 350 mg/L de FeSO₄, _{MnSO(4)} , MgSO₍₄) et/ou ZnSO₄ sont ajoutés au cours de l'étape (a) et/ou (b) du procédé.

11. Procédé selon l'une des revendications précédentes, dans lequel la cellule du champignon filamenteux est *Trichoderma.*

12. Procédé selon l'une quelconque des revendications précédentes, dans lequel la cellule du champignon filamenteux comprend au moins une enzyme bêta-glucosidase hétérologue.

13. Cellule de champignon filamenteux dans laquelle la séquence du gène SEQ1 (SEQ ID NO:1) a été perturbée.

14. Cellule de champignon filamenteux selon la revendication 13, dans laquelle SEQ ID NO:1 a été perturbé par délétion, mutation, modification d'un promoteur ou de toute autre séquence régulatrice, génération d'un codon stop ou interférence ARN.

15. Cellule de champignon filamenteux selon l'une quelconque des revendications 13 ou 14, dans laquelle la au moins une cellule de champignon filamenteux est une cellule de champignon filamenteux génétiquement modifiée ayant la capacité d'exprimer au moins une enzyme hydrolase hétérologue, au moins une enzyme pectinase hétérologue, au moins une enzyme oxydative hétérologue et/ou au moins une protéine accessoire hétérologue .

16. Cellule de champignon filamenteux selon l'une des revendications 13 à 15, dans laquelle l'au moins une cellule de champignon filamenteux est une cellule de champignon filamenteux génétiquement modifiée comprenant au moins une séquence codante d'enzyme bêta-glucosidase hétérologue, au moins une séquence codante d'enzyme bêta-xylosidase hétérologue, au moins une séquence d'encodage de l'enzyme xylanase hétérologue, au moins une séquence d'encodage de l'enzyme pectinase hétérologue, au moins une séquence d'encodage de l'enzyme lytique polysaccharide monooxygénase hétérologue, au moins une séquence d'encodage de l'enzyme oxydative hétérologue et/ou au moins une séquence d'encodage de la protéine accessoire hétérologue.

17. Composition enzymatique de bouillon entier produite selon un procédé tel que défini dans l'une des revendications 1 à 12.

18. Utilisation d'une cellule de champignon filamenteux telle que définie dans l'une des revendications 13 à 16 pour la production d'une composition enzymatique de bouillon entier.

19. Utilisation d'une composition enzymatique de bouillon entier selon la revendication 18 pour l'hydrolyse de la biomasse lignocellulosique.
